(19) Europäisches Patentamt / European Patent Office / Office européen des brevets

(11) **EP 4 810 625 A1**

(12) **EUROPEAN PATENT APPLICATION**


(43) Date of publication:
**23.09.2026 Bulletin 2026/39**

(21) Application number: **26179466.3**

(22) Date of filing: **23.06.2022**

(51) International Patent Classification (IPC):
***C12Q 1/6806** (2018.01)* ***C12N 15/10** (2006.01)*
***C12Q 1/6834** (2018.01)*

(52) Cooperative Patent Classification (CPC):
(C-Sets available)
**C12Q 1/6806; C12N 15/1065; C12Q 1/6834**
(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.06.2021 US 202163214693 P**

(62) Document number(s) of the earlier application(s) in accordance with Art. 76 EPC:
**22829309.8 / 4 359 557**



(71) Applicant: **Illumina, Inc.**
**San Diego, CA 92122 (US)**

(72) Inventors:
- **MANZO, Andrea**
**California 92122 (US)**
- **BROWN, Colin**
**California 92122 (US)**
- **NORBERG, Steven**
**California 92122 (US)**
- **HARRINGTON, Timothy**
**California 92122 (US)**

(74) Representative: **Williams, Andrea**
**Marks & Clerk LLP**
**62-68 Hills Road**
**Cambridge CB2 1LA (GB)**



Remarks:
•The complete document including Reference Table(s) and the Sequence Listing(s) can be downloaded from the EPO website
•This application was filed on 19-05-2026 as a divisional application to the application mentioned under INID code 62.
•Claims filed after the date of filing of the application/ after the date of receipt of the divisional application (Rule 68(4) EPC).


(54) **METHODS AND COMPOSITIONS FOR COMBINATORIAL INDEXING OF BEAD-BASED NUCLEIC ACIDS**


(57) Some embodiments relate to methods and compositions for preparing combinatorially indexed beads. Some embodiments include sequential addition of different indexes to polynucleotides attached to beads. In some embodiments, indexes are added by chemical ligation, polymerase extension, ligation of partially double-stranded adaptors, or short splint ligation.

Step 1:
Link oligo to bead through Bio-SA
Each well receives SA-beads and a unique oligo sequence (Index 1)
Step 2:
Pool all beads
Distribute bead pool across all wells
Step 3:
Attach oligo to bead-linked oligo
Each well receives a unique oligo sequence (Index B)
Step 4:
Pool all beads
Distribute pool across all wells
Step 5:
Ligate oligo to bead-linked oligo
Each well receives a unique oligo sequence (Index C)
Step 6:
Pool all beads
P5
Index 1
Link 1
Index 2
Link 2
Index 3
Hyb


FIG. 1

(52) Cooperative Patent Classification (CPC): (Cont.)

C-Sets
**C12Q 1/6806, C12Q 2521/131, C12Q 2523/109, C12Q 2525/117, C12Q 2525/197, C12Q 2525/191, C12Q 2563/149, C12Q 2563/179, C12Q 2563/131; C12Q 1/6834, C12Q 2521/101, C12Q 2521/501, C12Q 2525/191, C12Q 2537/149, C12Q 2563/149, C12Q 2563/179**

**Description**

RELATED APPLICATIONS

This application claims priority to U.S. Prov. No. 63/214,693 filed June 24, 2021 entitled "METHODS AND COMPOSITIONS FOR COMBINATORIAL INDEXING OF BEAD-BASED NUCLEIC ACIDS" which is incorporated by reference in its entirety.

REFERENCE TO SEQUENCE LISTING

The present application is being filed along with a Sequence Listing in electronic format. The Sequence Listing is provided as a file entitled ILLINC608WOSEQLIST, created June 17, 2022, which is approximately 3.15 kilobytes in size. The information in the electronic format of the Sequence Listing is incorporated herein by reference in its entirety.

FIELD OF THE INVENTION

Some embodiments relate to methods and compositions for preparing combinatorially indexed beads. Some embodiments include sequential addition of different indexes to polynucleotides attached to beads. In some embodiments, indexes are added by chemical ligation, polymerase extension, ligation of partially double-stranded adaptors, or short splint ligation.

BACKGROUND OF THE INVENTION

The detection of specific nucleic acid sequences present in a biological sample has been used, for example, as a method for identifying and classifying microorganisms, diagnosing infectious diseases, detecting and characterizing genetic abnormalities, identifying genetic changes associated with cancer, studying genetic susceptibility to disease, and measuring response to various types of treatment. A common technique for detecting specific nucleic acid sequences in a biological sample is nucleic acid sequencing.

Nucleic acid sequencing methodology has evolved significantly from the chemical degradation methods used by Maxam and Gilbert and the strand elongation methods used by Sanger. Several sequencing methodologies are now in use which allow for the parallel processing of thousands of nucleic acids all on a single chip. Some platforms include bead-based and microarray formats in which silica beads are functionalized with probes depending on the application of such formats in applications including sequencing, genotyping, gene expression profiling.

Methods of genotyping different samples on current bead-based arrays can require gaskets to physically subdivide different areas of bead chips into multiple sectors. Individual samples are then loaded into each discrete section created by the gasket. However, such methods might be used with relatively low sample number input but prove arduous or unmanageable as the density of samples per bead chip increase from 24 to 96, 384, 1536, or more samples per bead chip.

SUMMARY OF THE INVENTION

Some embodiments of the methods and compositions provided herein include methods of preparing a plurality of combinatorially indexed beads, comprising: (a) obtaining a population of primary indexed beads comprising a first polynucleotide comprising a first index, wherein the population of primary indexed beads comprises a plurality of first subpopulations of indexed beads, wherein the first subpopulations of indexed beads comprise a different first index from one another; (b) splitting the population of primary indexed beads into a plurality of second subpopulations of beads; (c) obtaining a population of secondary indexed beads, comprising: (i) extending the first polynucleotide of the plurality of second subpopulations of beads with a second polynucleotide comprising a second index to obtain second subpopulations of indexed beads, wherein the second subpopulations of indexed beads comprise a different second index from one another, and (ii) combining the second subpopulations of indexed beads to obtain the population of secondary indexed beads; and optionally, further comprising: (d) splitting the population of secondary indexed beads into a plurality of third subpopulations of beads; and (e) obtaining a population of tertiary indexed beads, comprising: (i) extending the second polynucleotide of the plurality of third subpopulations of beads with a third polynucleotide comprising a third index to obtain third subpopulations of indexed beads, wherein the third subpopulations of indexed beads comprise a different third index from one another, and (ii) combining the third subpopulations of indexed beads to obtain the population of tertiary indexed beads.

In some embodiments, (c) comprises extending the first polynucleotide with the second polynucleotide by chemical ligation.

In some embodiments, the first polynucleotide comprises a terminal 3’ modified deoxynucleotide (dNTP)

comprising a 3' functional moiety capable of participating in a click chemistry reaction; the second polynucleotide comprises a terminal 5' modified dNTP comprising a compatible 5' functional moiety capable of participating in a click chemistry reaction with the 3'-functional moiety; and the 3' functional moiety and the 5' functional moiety are capable of reacting with one another to form a modified backbone linkage.

In some embodiments, the extending the first polynucleotide with the second polynucleotide obtains a secondary indexed polynucleotide, and the method further comprises modifying the secondary indexed polynucleotide to obtain a modified polynucleotide comprising a terminal 3' modified deoxynucleotide (dNTP) comprising a 3' functional moiety capable of participating in a click chemistry reaction.

In some embodiments, the modifying comprises contacting the secondary indexed polynucleotide with a template independent polymerase. In some embodiments, the template independent polymerase is selected from a terminal deoxynucleotidyl transferase (TdT), PolyA polymerase, or CCA-adding RNA polymerase. In some embodiments, the template independent polymerase is TdT.

Some embodiments also include extending the modified polynucleotide with the third polynucleotide by a chemical ligation reaction, wherein the third polynucleotide comprises a terminal 5' modified dNTP comprising a compatible 5' functional moiety capable of participating in a click chemistry reaction with the 3' functional moiety.

In some embodiments, the 3' functional moiety is selected from the group consisting of an azide, an alkynyl, an alkenyl, a thiol, and a nitrone.

In some embodiments, the 5' functional moiety is different from and compatible with the 3' functional moiety and is selected from the group consisting of an azide, an alkynyl, an alkenyl, a thiol, and a nitrone

In some embodiments, the 3' functional moiety and the 5' functional moiety are selected from the following pairs: (i) 3'-azido/5'-alkynyl; (ii) 3'-alkynyl/5' azido; (iii) 3'-thiol/5'-alkynyl; (iv) 3'-thiol/5'-alkenyl; (v) 3'-alkynyl/5'-thiol; (vi) 3'-alkenyl/5'-thiol; (vii) 3'-azido/5'-cyclooctynyl; (viii) 3'-cyclooctyne/5'-azido; (ix) 3'-nitrone/5'-cyclooctynyl; and (x) 3'-cyclooctynyl/5'-nitrone. In some embodiments, the 3' functional moiety is a 3'-azido and the 5' functional moiety is a 5'-alkynyl.

In some embodiments, the click chemistry reaction comprises copper catalyzed azide-alkyne cycloaddition (CuAAC) to form a modified backbone linkage comprising a triazolyl.

In some embodiments, (c) comprises extending the first polynucleotide by polymerase extension, wherein the first polynucleotide comprises a first linker.

Some embodiments also include (i) obtaining a first adaptor comprising a region capable of hybridizing to the first linker and a region comprising the second index or complement of the second index; (ii) hybridizing the first adaptor to the first linker; and (iii) extending the first polynucleotide to obtain a secondary indexed polynucleotide.

In some embodiments, the first adaptor comprises a non-extendable 3' end. In some embodiments, the non-extendable 3' end comprises a 3'2' dideoxy nucleotide, or a C3 linker.

Some embodiments also include removing the first adaptor from the secondary indexed polynucleotide. In some embodiments, the removing comprises denaturing the first adaptor by heat or base, or degrading the first adaptor by enzymic degradation.

In some embodiments, the first linker has a length less than 10 consecutive nucleotides. In some embodiments, the first linker has a length less than 5 consecutive nucleotides.

In some embodiments, (e) comprises extending the second polynucleotide by polymerase extension.

In some embodiments, the first adaptor comprises a complement of a second linker, such that the secondary indexed polynucleotide comprises the second linker.

Some embodiments also include (i) obtaining a second adaptor comprising a region capable of hybridizing to the second linker and a region comprising the third index or complement of the third index; (ii) hybridizing the second adaptor to the second linker; and (iii) extending the secondary indexed polynucleotide to obtain a tertiary indexed polynucleotide.

In some embodiments, the second adaptor comprises a non-extendable 3' end.

In some embodiments, the non-extendable 3' end comprises a 3'2' dideoxy nucleotide, or a C3 linker.

Some embodiments also include removing the second adaptor from the tertiary indexed polynucleotide. In some embodiments, the removing comprises denaturing the first adaptor by heat or base, or degrading the first adaptor by enzymic degradation.

In some embodiments, the second linker has a length less than 10 consecutive nucleotides. In some embodiments, the second linker has a length less than 5 consecutive nucleotides.

In some embodiments, (c) comprises extending the first polynucleotide with the second polynucleotide by ligation.

Some embodiments also include (i) obtaining a double stranded first adaptor comprising the second polynucleotide and a 3' single stranded overhang capable of hybridizing to a first linker of the first polynucleotide; (ii) hybridizing the first adaptor to the first linker; and (iii) ligating the first polynucleotide to the second polynucleotide to obtain a secondary indexed polynucleotide.

Some embodiments also include extending the second polynucleotide with a third polynucleotide by ligation.

Some embodiments also include (i) obtaining a double stranded second adaptor comprising the third polynucleotide and a 3' single stranded overhang capable of hybridizing to a second linker of the second polynucleotide; (ii) hybridizing the second adaptor to the second linker and optionally, hybridizing an additional oligonucleotide to the first index; and (iii) ligating the second polynucleotide to the third polynucleotide to obtain a tertiary indexed polynucleotide.

In some embodiments, the ligation comprises use of a ligase.

In some embodiments, the ligation comprises a chemical ligation reaction.

In some embodiments, (c) comprises extending the first polynucleotide by ligation, wherein the first polynucleotide comprises a first linker, and the second polynucleotide comprises a second linker.

Some embodiments also include (i) obtaining a first adaptor comprising a region capable of hybridizing to the first linker and a region capable of hybridizing to the second linker; (ii) hybridizing the first adaptor to the first linker; (iii) hybridizing the second oligonucleotide to the region capable of hybridizing to the second linker; and (iv) ligating the first polynucleotide to the second polynucleotide to obtain a secondary indexed polynucleotide.

In some embodiments, the first linker and/or the second linker has a length less than 10 consecutive nucleotides. In some embodiments, the first linker and/or the second linker has a length less than 5 consecutive nucleotides.

In some embodiments, the first linker and/or the second linker is modified to have an increased Tm compared to an oligonucleotide having the same length as the first adaptor.

In some embodiments, the first linker and/or the second linker comprises an increased G/C content compared to the oligonucleotide having the same length, or comprises modified nucleotides.

Some embodiments also include removing the first adaptor from the secondary indexed polynucleotide. In some embodiments, the removing comprises denaturing the first adaptor by heat or base, or degrading the first adaptor by enzymic degradation.

In some embodiments, (e) comprises extending the second polynucleotide by ligation, wherein the second oligonucleotide comprises a third linker, such that the secondary indexed polynucleotide comprises the third linker, and wherein the third polynucleotide comprises a fourth linker.

Some embodiments also include (i) obtaining a second adaptor comprising a region capable of hybridizing to the third linker and a region capable of hybridizing to the fourth linker; (ii) hybridizing the second adaptor to the third linker; (iii) hybridizing the third polynucleotide to the second adaptor via the region capable of hybridizing to the fourth index; and (iv) ligating the secondary indexed polynucleotide to the third polynucleotide to obtain a tertiary indexed polynucleotide.

In some embodiments, the third linker and/or the fourth linker has a length less than 9 consecutive nucleotides. In some embodiments, the third linker and/or the fourth linker has a length less than 5 consecutive nucleotides.

In some embodiments, the third linker and/or the fourth linker is modified to have an increased Tm compared to an oligonucleotide having the same length as the second adaptor.

In some embodiments, the third linker and/or the fourth linker comprises an increased G/C content compared to the oligonucleotide having the same length, or comprises modified nucleotides.

Some embodiments also include removing the second adaptor from the tertiary indexed polynucleotide.

In some embodiments, the removing comprises denaturing the first adaptor by heat or base, or degrading the first adaptor by enzymic degradation.

In some embodiments, (a) comprises: (i) attaching the first polynucleotide to a plurality of first subpopulations of beads to obtain the first subpopulations of indexed beads, wherein the first polynucleotide comprises a different first index for each first subpopulation of beads; and (ii) combining the first subpopulations of indexed beads to obtain the population of primary indexed beads.

In some embodiments, the first polynucleotide is attached to a bead via a first binding partner and a second binding partner. In some embodiments, the first binding partner or the second binding partner is selected from the group consisting of biotin, streptavidin, a biotin derivative, a streptavidin derivative, an antibody, and an antigen binding fragment of an antibody.

Some embodiments also repeating (d) and (e) and adding additional indexes to indexed subpopulations of beads.

In some embodiments, the first polynucleotide comprises a primer binding site selected from the group consisting of a P5 sequence, a P5' sequence, P7 sequence, and P7' sequence.

In some embodiments, the first index, the second index, and/or the third index has a length less than 20 consecutive nucleotides. In some embodiments, the first index, the second index, and/or the third index has a length less than 10 consecutive nucleotides.

In some embodiments, (b) comprises randomly distributing the population of primary indexed beads into a plurality of compartments.

In some embodiments, (d) comprises randomly distributing the population of secondary indexed beads into a plurality of compartments.

In some embodiments, the plurality of compartments comprise a compartment selected from a well, a channel, or a droplet.

In some embodiments, the plurality of combinatorially indexed beads comprise magnetic beads.

Some embodiments also distributing the plurality of combinatorially indexed beads on an array. Some embodiments also sequencing the combinatorially indexed beads on an array.

Some embodiments also decoding the location of a bead of the plurality of combinatorially indexed beads on an array based on the combinatorial indexes.

In some embodiments, each bead of the plurality of combinatorially indexed beads comprises a capture probe. In some embodiments, the first polynucleotide, the second polynucleotide or the third polynucleotide comprises the capture probe. Some embodiments also hybridizing a plurality of target nucleic acid to the capture probes. Some embodiments also extending the capture probes.

## BRIEF DESCRIPTION OF THE DRAWINGS

FIG. 1 depicts an example embodiment of combinatorial indexing including a pool and split strategy.

FIG. 2 depicts an embodiment of a scheme to join to a first index (index A) attached to a substrate, a second index (index B), and a third index (index C) by sequential splinted ligation reactions with linker-splints.

FIG. 3 depicts an embodiment of a scheme to join a second index (index B) to a first index (index A) attached to a substrate by click chemistry ligation.

FIG. 4 depicts an embodiment of a scheme to add to a first index (index A) attached to a substrate, a second index (index B) and a third index (index C) by sequential polymerase extension reaction.

FIG. 5 depicts an embodiment of a scheme to add to a first index (index A) attached to a substrate, a second index (index B) and a third index (index C) using an adaptor comprising a double-stranded region and single-stranded overhang.

FIG. 6 depicts an embodiment of a scheme to add to a first index (index A) attached to a substrate, a second index (index B) and a third index (index C) by sequential splinted ligation reactions with short linker-splints.

FIG. 7A depicts a schematic including a bead with a capture oligonucleotide (P5-index A-Link 1a) hybridized with an extension template (Link 1a'-index B'-Hyb') for polymerase extension, and the positions of primers useful to measure amounts of capture oligonucleotide; and a bead with an extension product, and the positions of primers useful to measure amounts of full length extension products.

FIG. 7B depicts a graph for amounts of capture oligonucleotides and of full length product from ligation extension of capture oligonucleotides or polymerase extension of capture oligonucleotides under various conditions.

FIG. 8A is a schematic outlining steps and conditions tested in methods for 3-level indexing that include extension of capture oligonucleotides by either polymerase extension or ligation. The numbering corresponds to conditions tested.

FIG. 8B is a table which summarizes various experimental conditions to compare splint ligation (SL) and polymerase extension (PE), and includes various conditions (cond) numbered in FIG. 8A for level 1 indexing (L1), and level 2 indexing (L2), and level 3 indexing (L3).

FIG 9 is a graph showing amounts of capture oligonucleotides (CO), 2nd level extension products, and 3rd level extension products under various conditions.

FIG. 10 is a graph of the concentration of capture oligonucleotides with a 1st-extension product attached to beads via biotin or dual desthiobiotin (ddbiotin) and treated under various denaturing conditions, as measured by quantative PCR.

FIG. 11A is a schematic showing sequencing read orientation for a synthesis product on a bead including a 3-level indexing product, a PCR product derived from the synthesis product, and a sequencing read derived from the PCR product.

FIG. 11B is a graph showing percentage of sequencing reads that include sequences that are either fully correct or where both indexes are usable for extension products generated by polymerase extension or splint ligation. Useable indexes include those that could be decoded corrected with an index error correction implementation, and included no more than one 'SNP' within indexes designed with 3 nucleotide Hamming distance, and no indels across entire 'index' region, for example, in a 3-level index oligonucleotide, the index region includes 3 indexes.

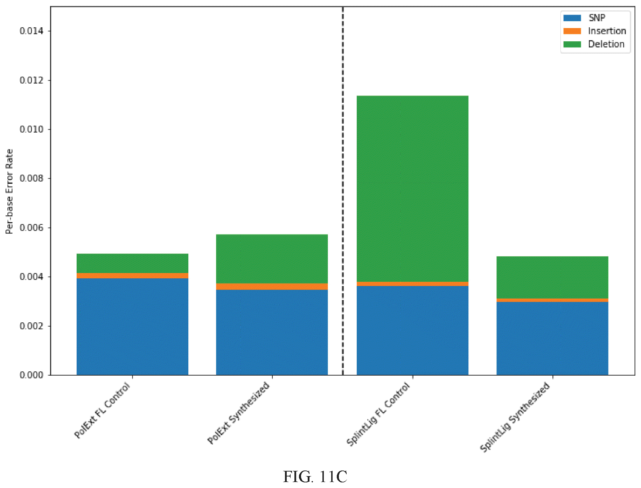

FIG. 11C is a graph showing per base error rate including whether the error is a deletion, insertion or base change (SNP), for sequencing reads that include sequences that are either fully correct or where both indexes are usable for extension products generated by polymerase extension or splint ligation. Useable indexes include those that could be decoded corrected with an index error correction implementation, and included no more than one 'SNP' within indexes designed with 3 nucleotide Hamming distance, and no indels across entire 'index' region, for example, in a 3-level index oligonucleotide, the index region includes 3 indexes.

FIG. 12A is a schematic showing steps in a standard splint ligation method (left panel), and a double-stranded splint ligation method (right panel). In some embodiments, the double-stranded splint ligation method can include an

additional oligonucleotide (index1').

FIG. 12B is a schematic showing conditions tested in steps in methods for a standard splint ligation and a double-stranded splint ligation.

FIG. 12C is a schematic showing positions for forward or reverse primers to measure extension products from either standard ligation or double-stranded ligation.

FIG. 13A is a graph showing concentration of extension products, as measured by quantitative PCR. Capture oligonucleotides were measured using F2 and R1 primers, and full-length products were measured using either F2 and R2 or F3 and R2 primers as depicted in FIG. 12C.

FIG. 13B is a graph showing relative concentration of extension products normalized to full length control.

FIG. 13C is a graph showing relative concentration of extension products normalized to capture oligonucleotide concentration.

FIG. 13D is a graph showing concentration of extension products generated in the presence of various amounts of double-stranded splint oligo.

FIG. 13E is a graph showing concentration of extension products generated in the presence of an additional oligonucleotide complementary to index1, as shown in FIG. 12A.

FIG. 13F is a graph showing concentration of extension products generated in the presence of an oligonucleotide having a non-extendable 3'ddC end.

FIG. 13G is a graph showing concentration of extension products generated with a pre-ligation step at room temperature or 75°C.

FIG. 14A is a graph showing percentage of sequencing reads that include sequences that are either fully correct or where both indexes are usable for extension products generated by splint ligation extension or double-stranded splint ligation. Useable indexes include those that could be decoded corrected with an index error correction implementation, and included no more than one 'SNP' within indexes designed with 3 nucleotide Hamming distance, and no indels across entire 'index' region for example, in a 3-level index oligonucleotide, the index region includes 3 indexes.

FIG. 14B is a graph showing per base error rate including whether the error is a deletion, insertion or base change (SNP), for sequencing reads for extension products generated by splint ligation extension or double-stranded splint ligation.

FIG. 15A is a schematic showing steps in 3-level indexing for a standard splint ligation method (left panel), and a double-stranded splint ligation method (right panel).

FIG. 15B is a schematic showing conditions tested in steps in methods for 3-level indexing with a standard splint ligation or a double-stranded splint ligation.

FIG. 16A is a graph showing concentration of extension products for capture oligonucleotides, 2nd-level products, and 3rd level products, as measured by quantitative PCR.

FIG. 16B is a graph showing percentage of sequencing reads that include sequences that are either fully correct or where all three indexes are usable for extension products generated by splint ligation extension or double-stranded splint ligation. Useable indexes include those that could be decoded corrected with an index error correction implementation, and included no more than one 'SNP' within indexes designed with 3 nucleotide Hamming distance, and no indels across entire 'index' region, for example, in a 3-level index oligonucleotide, the index region includes 3 indexes.

FIG. 16C is a graph showing per base error rate including whether the error is a deletion, insertion or base change (SNP), for sequencing reads for extension products generated by splint ligation extension or double-stranded splint ligation.

FIG. 17 is a schematic showing extension of capture oligonucleotides by standard splint ligation, or by splint ligation using a shortened splint (upper panel). Lower panel depicts the positions of primers to measure extension products.

FIG. 18A is a schematic showing various splint oligonucleotides. The sequences depicted in FIG. 18A include SEQ ID NOs:07-16.

FIG. 18B is a table showing various experimental conditions including a capture oligonucleotide with level 1 index (L1 oligo), a splint oligonucleotide (splint), and a level 2 oligonucleotide (L2 oligo).

FIG. 18C is a graph of full-length extension products relative to control full length extension products for extension products generated by splint ligation extension with various splint oligonucleotides.

FIG. 19A is a schematic for a combinatorial indexing method including double-stranded splint ligation which includes linker sequences "KS-3'" and "MS-3'".

FIG. 19B is a schematic for an overview of an example workflow for a combinatorial indexing method including double-stranded splint ligation.

## DETAILED DESCRIPTION

Some embodiments relate to methods and compositions for preparing combinatorially indexed beads. Some

embodiments include sequential addition of different indexes to polynucleotides attached to beads. In some embodiments, indexes are added by chemical ligation, polymerase extension, ligation of partially double-stranded adaptors, or short splint ligation.

Pools of bead-linked oligonucleotides in which each bead is uniformly coated with a single oligonucleotide sequence are a component of sequencing methods such as synthetic long read sequencing. Combinatorial assembly of such bead pools can be used to achieve sufficient index diversity; however some methods rely on a splinted ligation strategy to join successive levels of the index. Splint ligation has the disadvantage of introducing invariant bases into the bead code and increasing the number of sequencing by synthesis (SBS) cycles required to read a complete bead code. Certain embodiments provided herein include several alternatives to the standard splint ligation bead code synthesis strategy that reduce the number of invariant bases in the bead code, increasing bead code information density and allowing for more efficient bead code sequencing.

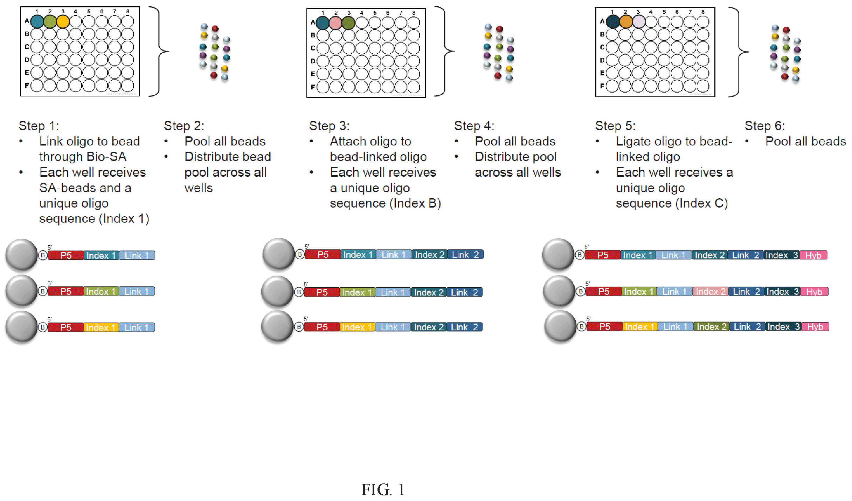

Combinatorial assembly of bead-linked oligonucleotides allows for the generation of bead pools containing large numbers of unique indexes in which each bead is uniformly coated with a single, unique index oligonucleotide. Starting with a common pool of magnetic beads derivatized for oligonucleotide capture, such as surface-attached streptavidin, beads are aliquoted into 'M' wells of a multiwell plate, each well containing a single oligonucleotide sequence that has been synthesized with a bead-capture moiety, such as biotin. After binding, the beads are again pooled, mixed, and split into 'N' wells of a multiwell plate to attach a second-level index. Each well in the second index reaction contains a uniform mixture of first-level oligonucleotides, so the total number of unique indexes becomes 'M' x 'N' after the second index is attached. Repeating this process multiple times can yield bead pools with millions of unique indexes from small sets of individual index oligonucleotides that can easily fit in standard multiwell plates, such as 96- 192- or 384-well plates. An example embodiment of combinatorial indexing including a pool and split strategy is depicted in FIG. 1.

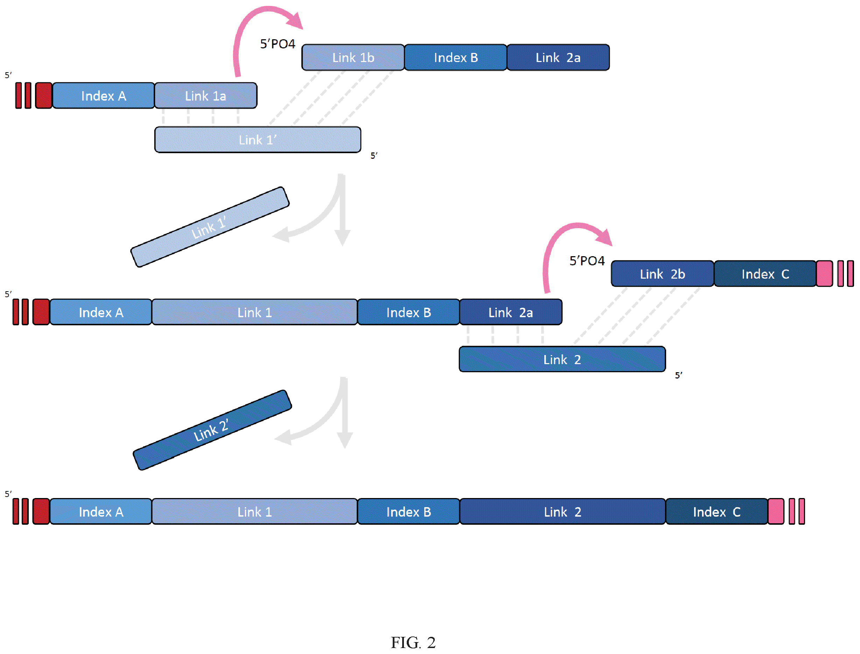

For indexes beyond a directly captured first level, a method is needed to covalently attach single-stranded index oligonucleotides to the bead-bound indexes. This can be performed using a long splint ligation approach (FIG. 2). The directly captured first-level oligonucleotide is designed to have an 8 nucleotide capture sequence (L1A) at its 3' end, and the incoming index oligonucleotide has a second 8 nucleotide capture sequence (L1B) on its 5' end, as well as a phosphate on the 5' end. In some embodiments, the LIA and/or LIB can have a length equal to or greater than 4, 5, 6, 7, 8, 9, 10, 11, 12, 13, 14, 15, 16, 17, 18, 19, or 20 nucleotides. These two pieces are then assembled in a ligation reaction with a 16 nucleotides splint oligonucleotide that is complementary to L1A at the 3' end and L1B at the 5' end; the bound splint hybridizes with both the bead-bound capture oligonucleotide and the in-solution index oligonucleotide bringing the 3' and 5' ends of the two pieces directly adjacent to one another. A ligase enzyme in the reaction then covalently links the phosphorylated 5' end of the index oligonucleotide to the 3' end of the capture oligo. TABLE 1 lists the number of unique bead codes that may be obtained using 12-, 96, 192-, or 384- and 768- multiwell plates in certain methods. Some such methods include: (a) distributing beads in wells of a 1$^{st}$ multiwell plate, each well contains a different index A; attaching index A to the beads; pooling the beads; (b) redistributing the beads in wells of a 2$^{nd}$ multiwell plate, each well contains a different index B, and attaching index B to the beads; (c) redistributing the beads in wells of a 3$^{rd}$ multiwell plate, each well contains a different index C, and attaching index C to the beads.

TABLE 1

| **Index C** | **Index B** | **Index A** | | | |
|---|---|---|---|---|---|
| | | **12-well plate** | **96-well plate** | **192-well plate** | **384-well plate** |
| **96-well plate** | **96-well plate** | 110,592 | 884,736 | 1,769,472 | 3,538,944 |
| | **192-well plate** | 221,184 | 1,769,472 | 3,538,944 | 7,077,888 |
| | **384-well plate** | 442,368 | 3,538,944 | 7,077,888 | 14,155,776 |
| | **768-well plate** | 884,736 | 7,077,888 | 14,155,776 | 28,311,552 |
| **192-well plate** | **96-well plate** | 221,184 | 1,769,472 | 3,538,944 | 7,077,888 |
| | **192-well plate** | 442,368 | 3,538,944 | 7,077,888 | 14,155,776 |
| | **384-well plate** | 884,736 | 7,077,888 | 14,155,776 | 28,311,552 |
| | **768-well plate** | 1,769,472 | 14,155,776 | 28,311,552 | 56,623,104 |
| **384-well plate** | **96-well plate** | 221,184 | 1,769,472 | 3,538,944 | 7,077,888 |
| | **192-well plate** | 442,368 | 3,538,944 | 7,077,888 | 14,155,776 |
| | **38-well plate 4** | 884,736 | 7,077,888 | 14,155,776 | 28,311,552 |
| | **768-well plate** | 1,769,472 | 14,155,776 | 28,311,552 | 56,623,104 |

The splinted ligation approach requires a 16 nucleotides invariant splint sequence between the variable index regions for each level of indexes in the bead code. Because the bead code is typically sequenced with a single read, these invariant regions must be read through with chemistry-only, such as 'dark' cycles during SBS. For example, each base in the invariant region requires an additional SBS cycle to maintain correct phasing. These are typically 'chemistry-only' or 'dark' SBS cycles in which the polymerization chemistry step is used without an imaging step. For a three level index with 8 nucleotides individual sub-indexes, this means that at least 32 total dark cycles are needed to sequence just 24 nucleotides of informative index sequence. This large number of cycles has been observed to result in decreased read quality, both for the index sequences and any subsequent insert sequencing reads.

Certain embodiments provided herein include bead code synthesis strategies in addition to the long splint ligation strategy to reduce the number of invariant bases, and consequently chemistry-only SBS cycles needed to read a bead code. Some such embodiments include bead code synthesis by chemical ligation using azide-alkyne click chemistry; bead code synthesis by templated polymerase extension; bead code synthesis by enzymatic ligation of double-stranded fragments; and bead code synthesis by splinted ligation with short splints containing nonstandard nucleotides.

Some embodiments provided herein in relate to high-throughput genotyping on arrays. Some embodiments relate to decoding the locations of microfeatures in an array. In some embodiments, microfeatures comprise polynucleotides having barcodes and indexes. Some embodiments include sequencing barcodes and indexes to identify the locations of polynucleotides in an array. Certain aspects that may be useful with the methods and compositions disclosed herein are disclosed in WO 2020/086746 which is incorporated by reference in its entirety.

Decoding by hybridization includes identifying the location of a capture probe in a randomly distributed array of capture probes. The method typically involves several successive cycles of hybridizing labeled hybridization probes to one or more portion of the capture probe, imaging hybridization events, and removing the hybridization probes. Decoding by hybridization requires specialized reagents, specialized fluidic devices and specialized detectors. In some embodiments, decoding by hybridization can take up to 8 hours with 7-8 successive cycles.

Embodiments provided herein include random-distributed arrays of polynucleotides comprising a primer binding site and a barcode. In some embodiments, the barcode can be readily sequenced to decode the array using a high throughput sequencing system. Some embodiments can significantly reduce the time taken to decode an array with no additional reagents, hybridization probes, or specialized decoding equipment.

Some embodiments include the use of next generation sequencing (NGS) techniques and bead-based microarrays. Some such embodiments deliver high-performance, low-cost and high throughput genotyping assays that can be run on a generic NGS sequencing platform with minor modifications to substrates and reagents.

Some embodiments include conducting a genotyping assay in which a multiwell plate containing 'S' wells is loaded with S bead pools, each bead pool having a unique sample index, with each well containing 'N' unique bead types. After nucleic acid library generation from samples, such as treating a nucleic acid sample with steps including random primer amplification followed by enzymatic fragmentation and clean up, each sample library is added to an indexed well and allowed to hybridize to the capture probes. After hybridization is complete, a single base extension assay to probe the SNP of interest is executed by adding an incorporation mix that includes fluorescent nucleotides and an appropriate polymerase. At the end of the incorporation, all bead-capture samples in a plate are pooled and loaded into a flowcell. The flowcell can be plain or patterned, and the surface appropriately modified to support the immobilization of beads at the desired density. In some embodiments, upon bead immobilization, a SNP readout is performed which includes a single scan cycle to read the signal deriving from fluorescent incorporation at the SNP site. This cycle may include an SBS cycle on the instrument. A barcode readout is also performed which includes 12-20 SBS cycles, depending on bead pool plexity, to identify capture probe and position of a specific bead within the flowcell. In some embodiments, this step could be replaced by additional cycles of sequencing past the identified SNP. A sample index readout is also performed which includes 6-12 SBS cycles to read the sample index. In some embodiments, the entire on-flowcell assay can include less than about 30 SBS cycles and can be executed in less than 4 hours.

## Definitions

As used herein, "nucleic acid" is intended to be consistent with its use in the art and includes naturally occur ring nucleic acids or functional analogs thereof. Particularly useful functional analogs are capable of hybridizing to a nucleic acid in a sequence specific fashion or capable of being used as a template for replication of a particular nucleotide sequence. Naturally occurring nucleic acids generally have a backbone containing phosphodiester bonds. An analog structure can have an alternate backbone linkage including any of a variety of those known in the art. Naturally occurring nucleic acids generally have a deoxyribose sugar (e.g. found in deoxyribonucleic acid (DNA)) or a ribose sugar (e.g. found in ribonucleic acid (RNA)). A nucleic acid can contain any of a variety of analogs of these sugar moieties that are known in the art. A nucleic acid can include native or non-native bases. In this regard, a native deoxyribonucleic acid can have one or more bases selected from the group consisting of adenine, thymine, cytosine or guanine and a ribonucleic acid can have one or more bases selected from the group consisting of uracil, adenine, cytosine or guanine. Useful non-native bases that

can be included in a nucleic acid are known in the art. Examples of non-native bases include a locked nucleic acid (LNA) and a bridged nucleic acid (BNA). LNA and BNA bases can be incorporated into a DNA oligonucleotide and increase oligonucleotide hybridization strength and specificity. LNA and BNA bases and the uses of such bases are known to the person skilled in the art and are routine.

As used herein, the term "nucleotide analogs" refers to synthetic analogs having modified nucleotide base portions, modified pentose portions, and/or modified phosphate portions, and, in the case of polynucleotides, modified internucleotide linkages. Modified internucleotide linkages include phosphate analogs, analogs having achiral and uncharged intersubunit linkages, and uncharged morpholino-based polymers having achiral intersubunit linkages. Some internucleotide linkage analogs include morpholidate, acetal, and polyamide-linked heterocycles. Examples of phosphate analogs include but are not limited to phosphorothioate, phosphorodithioate, phosphoroselenoate, phosphorodiselenoate, phosphoroanilothioate, phosphoranilidate, phosphoramidate, boronophosphates, including associated counterions, e.g., H+, NH4+, Na+, if such counterions are present. Examples of modified nucleotide base portions include but are not limited to 5-methylcytosine (5mC); C-5-propynyl analogs, including but not limited to C-5 propynyl-C and C-5 propynyl-U; 2,6-diaminopurine, also known as 2-amino adenine or 2-amino-dA); hypoxanthine, pseudouridine, 2-thiopyrimidine, isocytosine (isoC), 5-methyl isoC, and isoguanine (isoG). Examples of modified pentose portions include but are not limited to, locked nucleic acid (LNA) analogs including without limitation Bz-A-LNA, 5-Me-Bz-C-LNA, dmf-G-LNA, and T-LNA, and 2'-or 3'-modifications where the 2'- or 3'-position is hydrogen, hydroxy, alkoxy (e.g., methoxy, methoxy-ethyl, --O-methyl, ethoxy, allyloxy, isopropoxy, butoxy, isobutoxy and phenoxy), azido, amino, alkylamino, fluoro, chloro, or bromo. Other examples include 2-aminopurine; 5-bromo du; deoxyUridine; deoxyInosine; hydroxymethyl dC; 5-methyl dC; 5-Nitroindole; 5-hydroxybutynl-2'-deoxyuridine; and 8-aza-7-deazaguanosine.

As used herein, "target" when used in reference to a nucleic acid, is intended as a semantic identifier for the nucleic acid in the context of a method or composition set forth herein and does not necessarily limit the structure or function of the nucleic acid beyond what is otherwise explicitly indicated. A target nucleic acid may be essentially any nucleic acid of known or unknown sequence. It may be, for example, a fragment of genomic DNA or cDNA. Sequencing may result in determination of the sequence of the whole, or a part of the target molecule. The targets can be derived from a primary nucleic acid sample, such as a nucleus or cell free sample. In one embodiment, the targets can be processed into templates suitable for amplification by the placement of universal sequences at the ends of each target fragment. The targets can also be obtained from a primary RNA sample by reverse transcription into cDNA.

As used herein, "universal" when used to describe a nucleotide sequence, refers to a region of sequence that is common to two or more nucleic acid molecules where the molecules also have regions of sequence that differ from each other. A universal sequence that is present in different members of a collection of molecules can allow capture of multiple different nucleic acids using a population of universal capture nucleic acids, e.g., capture oligonucleotides that are complementary to a portion of the universal sequence, e.g., a universal capture sequence. Non-limiting examples of universal capture sequences include sequences that are identical to or complementary to P5 and P7 primers. Similarly, a universal sequence present in different members of a collection of molecules can allow the amplification or replication (e.g., sequencing) of multiple different nucleic acids using a population of universal primers that are complementary to a portion of the universal sequence, e.g., a universal anchor sequence. A capture oligonucleotide or a universal primer therefore includes a sequence that can hybridize specifically to a universal sequence. Two universal sequences that hybridize are referred to as a universal binding pair. For instance, a capture oligonucleotide and a universal capture sequence that hybridize are a universal binding pair.

As used herein, "P5" and "P7" may be used when referring to primer sequences or primer binding sites. The terms "P5' " (P5 prime) and "P7' " (P7 prime) refer to the complement of P5 and P7, respectively. It will be understood that any suitable amplification primers can be used in the methods presented herein, and that the use of P5 and P7 are example embodiments only. Uses of amplification primers such as P5 and P7 on flowcells are known in the art, as exemplified by the disclosures of WO 2007/010251, WO 2006/064199, WO 2005/065814, WO 2015/106941, WO 1998/044151, and WO 2000/018957 which are each incorporated by reference in its entirety. For example, any suitable forward amplification primer, whether immobilized or in solution, can be useful in the methods presented herein for hybridization to a complementary sequence and amplification of a sequence. Similarly, any suitable reverse amplification primer, whether immobilized or in solution, can be useful in the methods presented herein for hybridization to a complementary sequence and amplification of a sequence. One of skill in the art will understand how to design and use primer sequences that are suitable for capture and/or amplification of nucleic acids as presented herein.

As used herein, "compartment" is intended to mean an area or volume that separates or isolates something from other things. Example compartments include vials, tubes, wells, droplets, boluses, beads, vessels, surface features, or areas or volumes separated by physical forces such as fluid flow, magnetism, electrical current or the like. In one embodiment, a compartment is a well of a multi-well plate, such as a 96- or 384-well plate.

As used herein, the term "primer" and its derivatives refer generally to any nucleic acid that can hybridize to a target sequence of interest. Typically, the primer functions as a substrate onto which nucleotides can be polymerized by a polymerase; in some embodiments, however, the primer can become incorporated into the synthesized nucleic acid

strand and provide a site to which another primer can hybridize to prime synthesis of a new strand that is complementary to the synthesized nucleic acid molecule. The primer can include any combination of nucleotides or analogs thereof. In some embodiments, the primer is a single-stranded oligonucleotide or polynucleotide. The terms "polynucleotide" and "oligonucleotide" are used interchangeably herein to refer to a polymeric form of nucleotides of any length, and may include ribonucleotides, deoxyribonucleotides, analogs thereof, or mixtures thereof. The terms should be understood to include, as equivalents, analogs of either DNA or RNA made from nucleotide analogs and to be applicable to single stranded (such as sense or antisense) and double stranded polynucleotides. The term as used herein also encompasses cDNA, that is complementary or copy DNA produced from an RNA template, for example by the action of reverse transcriptase. This term refers only to the primary structure of the molecule. Thus, the term includes triple-, double- and single-stranded deoxyribonucleic acid ("DNA"), as well as triple-, double- and single-stranded ribonucleic acid ("RNA").

As used herein, "adaptor" or "adapter" and their derivatives, e.g., universal adaptor, refers generally to any linear oligonucleotide which can be ligated to a nucleic acid molecule or form a splint in a ligation reaction. In some embodiments, the adaptor or a portion of the adaptor is substantially complementary or complementary to the 3' end or the 5' end of a target sequence, such as a linker region of a target nucleic acid. Generally, the adaptor can include any combination of nucleotides and/or nucleic acids. In some aspects, the adaptor can include one or more cleavable groups at one or more locations. In another aspect, the adaptor can include a sequence that is substantially identical, or substantially complementary, to at least a portion of a primer, for example a universal primer. In some embodiments, the adaptor can include a barcode or tag to assist with downstream error correction, identification or sequencing. The terms "adaptor" and "adapter" are used interchangeably.

As used herein, "array" refers to a population of sites that can be differentiated from each other according to relative location. Different molecules that are at different sites of an array can be differentiated from each other according to the locations of the sites in the array. An individual site of an array can include one or more molecules of a particular type. For example, a site can include a single target nucleic acid molecule having a particular sequence or a site can include several nucleic acid molecules having the same sequence (and/or complementary sequence, thereof). The sites of an array can be different features located on the same substrate. Exemplary features include without limitation, wells in a substrate, beads (or other particles) in or on a substrate, projections from a substrate, ridges on a substrate or channels in a substrate. The sites of an array can be separate substrates each bearing a different molecule. Different molecules attached to separate substrates can be identified according to the locations of the substrates on a surface to which the substrates are associated or according to the locations of the substrates in a liquid or gel. Example arrays in which separate substrates are located on a surface include those having beads in wells. In some embodiments, an array can be located on a flowcell.

Combinatorial indexing

Some embodiments of the methods and compositions provided herein include preparation of indexed beads by combinatorial indexing in which a first polynucleotide attached to a bead is extended by sequential addition of indexes. In some embodiments, the first polynucleotide, such as a single-stranded DNA polynucleotide, is extended with a second polynucleotide, such as a single-stranded DNA polynucleotide, comprising an index by various methods including by chemical ligation, by polymerase extension, by ligation of a double stranded adaptor comprising the second polynucleotide and a single-stranded overhang, or by splint ligation.

Some embodiments include split and pool indexing. For example, a first population of beads is split into a first plurality of subpopulations, and a different first index is added to each subpopulation by attaching a first polynucleotide comprising the first index to the beads, and the subpopulations are combined to obtain a second population of beads. The second population of beads is split into a second plurality of subpopulations, and a different second index is added to each subpopulation by extending the attached first polynucleotide with a polynucleotide comprising the second index, and the subpopulations are combined to obtain a third population of beads. The third population of beads is split into a third plurality of subpopulations, and a different third index is added to each subpopulation by extending the second polynucleotide with a polynucleotide comprising the third index, and the subpopulations are combined to obtain a fourth population of beads. The splitting of the populations into subpopulations and adding different indexes to each subpopulation can be repeated to generate even more diverse combinatorial indexes.

Some embodiments for preparing a plurality of combinatorially indexed beads include: (a) obtaining a population of primary indexed beads comprising a first polynucleotide comprising a first index, wherein the population of primary indexed beads comprises a plurality of first subpopulations of indexed beads, wherein the first subpopulations of indexed beads comprise a different first index from one another; (b) splitting the population of primary indexed beads into a plurality of second subpopulations of beads; (c) obtaining a population of secondary indexed beads, comprising: (i) extending the first polynucleotide of the plurality of second subpopulations of beads with a second polynucleotide comprising a second index to obtain second subpopulations of indexed beads, wherein the second subpopulations of indexed beads comprise a different second index from one another, and (ii) combining the second subpopulations of indexed beads to obtain the

population of secondary indexed beads.

Some embodiments also include: (d) splitting the population of secondary indexed beads into a plurality of third subpopulations of beads; and (e) obtaining a population of tertiary indexed beads, comprising: (i) extending the second polynucleotide of the plurality of third subpopulations of beads with a third polynucleotide comprising a third index to obtain third subpopulations of indexed beads, wherein the third subpopulations of indexed beads comprise a different third index from one another, and (ii) combining the third subpopulations of indexed beads to obtain the population of tertiary indexed beads. Some embodiments also repeating (d) and (e) and adding additional indexes to indexed subpopulations of beads to obtain even more diverse to generate even more diverse combinatorial indexes.

In some embodiments, obtaining the population of primary indexed beads comprising a first polynucleotide comprising a first index comprises: (i) attaching the first polynucleotide to a plurality of first subpopulations of beads to obtain the first subpopulations of indexed beads, wherein the first polynucleotide comprises a different first index for each first subpopulation of beads; and (ii) combining the first subpopulations of indexed beads to obtain the population of primary indexed beads. In some embodiments, the first polynucleotide is attached to a bead of the plurality of first subpopulations of beads via a first binding partner and a second binding partner. In some embodiments, the first binding partner or the second binding partner is selected from the group consisting of biotin, streptavidin, a biotin derivative, a streptavidin derivative, an antibody, and an antigen binding fragment of an antibody. In some embodiments, the first polynucleotide is attached to a bead by a covalent attachment, for example via a chemical reaction.

In some embodiments, the first polynucleotide comprises a primer binding site. Examples of primer binding sites can include a P5 sequence, a P5' sequence, P7 sequence, and P7' sequence. P5 sequence: AAT GAT ACG GCG ACC ACC GA (SEQ ID NO:01); and P7 sequence: CAA GCA GAA GAC GGC ATA CGA GAT (SEQ ID NO:02). In some embodiments, the first polynucleotide comprises a cleavable linker. In some embodiments, the first polynucleotide comprises a universal sequence.

In some embodiments, the first index, the second index, and/or the third index has a length greater than, less than, or equal to 100, 90, 80, 70, 60, 50, 40, 30, 25, 20, 19, 18, 17, 16, 15, 14, 13, 12, 11, 10, 9, 8, 7, 6, 5, 4, 3, 2 consecutive nucleotides, or a length within a range of any two of the foregoing numbers. In some embodiments, an index or a combination of indexes can be used to tag an element such as an attached nucleic acids, or a bead. Certain methods and compositions useful with embodiments provided herein are disclosed in U.S. 20180023119 and U.S. 20180355348 which are each incorporated by reference in its entirety.

In some embodiments, splitting a population of indexed beads, such as a population of primary indexed beads or population of secondary indexed beads, can include randomly distributing the population of indexed beads into a plurality of compartments. In some embodiments, the plurality of compartments comprise a compartment selected from a well, a channel, or a droplet. In some embodiments, the plurality of compartments comprises a 96-well plate, a 192-well plate, or a 384-well plate. In some embodiments a flow cell comprises the plurality of compartments.

In some embodiments, the plurality beads comprise magnetic beads.

Some embodiments also include distributing a plurality of combinatorially indexed beads on an array. In some embodiments, the plurality of combinatorially indexed beads is randomly distributed on the array.

Some embodiments also include sequencing the combinatorially indexed beads. For example, the combinatorial index or a complement thereof of a bead can be sequenced. In some embodiments, the combinatorial index or a complement thereof can be sequenced on an array. In some embodiments, sequencing can include sequencing by synthesis (SBS). Example SBS procedures, fluidic systems and detection platforms that can be readily adapted for use with methods and compositions provided herein are described, for example, in Bentley et al., Nature 456:53-59 (2008), WO 04/018497; U.S. Pat. No. 7,057,026; U.S. Pat. No. 7,329,492; U.S. Pat. No. 7,211,414; U.S. Pat. No. 7,315,019; U.S. Pat. No. 7,405,281, each of which is incorporated herein by reference. Some embodiments also include decoding the location of a bead of the plurality of combinatorially indexed beads on an array based on the combinatorial index of the bead.

In some embodiments, a bead of the plurality of combinatorially indexed beads comprises a capture probe. In some embodiments, the first polynucleotide, the second polynucleotide or the third polynucleotide comprises the capture probe. Some embodiments also include hybridizing a plurality of target nucleic acid to the capture probes. Some embodiments also include extending the capture probes.

## Combinatorial indexing comprising chemical ligation

Some embodiments of the methods and compositions provided herein include preparation of indexed beads by combinatorial indexing in which a polynucleotide attached to a bead is extended by sequential addition of indexes with chemical ligation. Certain methods and compositions useful with embodiments provided herein are disclosed in U.S. 20180127816 which is incorporated by reference in its entirety.

In some embodiments, sequential addition of indexes by chemical ligation includes a Copper-catalyzed azide-alkyne cycloaddition "click" reaction (CuAAC) to covalently bond successive levels of index oligonucleotides or poly-

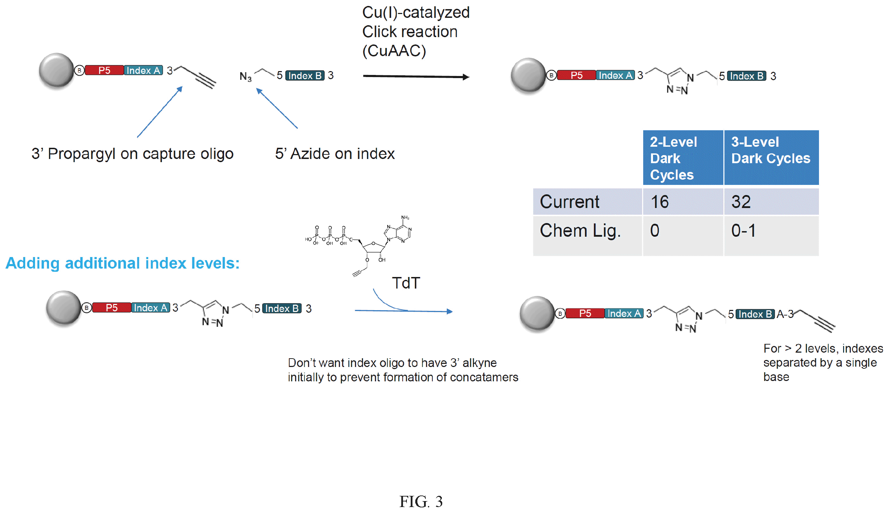

nucleotides (FIG. 3). Click chemistry is compatible with DNA oligonucleotides, and a cyclic triazole product has similar dimensions to the phosphodiester bond present in DNA. The similar dimensions of a cyclic triazole product to the phosphodiester bond present in DNA makes the triazole linkage a viable template for many DNA polymerases, and triazole-containing DNA strands can by amplified by methods including a polymerase chain reaction (PCR). An advantage of chemical ligation includes a method in which the ends of different single-stranded polynucleotides can be joined in the absence of a splint oligonucleotide or splint adaptor, by using an excess of index oligonucleotides or polynucleotides, and eliminating all or just a handful of invariant base positions within a final bead code or combinatorial index. In addition, the absence of an enzymatic ligation step increases flexibility in reaction conditions, as the CuAAC reaction can be carried out in alternative solvents and at nonstandard temperatures.

In some embodiments, chemical ligation for bead code or combinatorial index synthesis includes a two-step synthesis strategy. For the first level of the bead code, the initial capture oligonucleotide, such as a first polynucleotide comprising a first index, is produced with a CuAAC handle (azide or alkyne) at the 3' end, and this oligonucleotide is bound to magnetic beads via binding partners such as 5'-biotin / streptavidin. For the second level of the bead code, an excess of index oligonucleotide, such as a second polynucleotide comprising a second index, with a cognate CuAAC handle, such as 3'-Azide / 5' Alkyne or 3'-Alkyne / 5'-Azide, is added along with a copper catalyst to the bead-bound first polynucleotide. In some embodiments, the polynucleotide comprising a second index will only have the click handle on the 5' end. When the reaction is completed, the beads are pelleted and any remaining CuAAC reagents washed away. This results in an essentially 'scar-less' junction between the index levels, such as the first polynucleotide and second polynucleotide, with no invariant bases separating the levels.

In some embodiments, attaching a third polynucleotide comprising a third index can include the use of the second polynucleotide synthesized with complementary click handles on both the 5' and 3' ends; however, this could result in the formation of long concatemers. To add additional index levels while avoiding formation of concatemers, an enzyme such as a terminal deoxynucleotidyl transferase (TdT) is used to insert a single base, containing a click handle at the 3' OH position of deoxyribose, to the 3' end of the assembled two-level bead code. TdT attaches nucleotide triphosphates to the 3' end of a single-stranded oligonucleotide with little specificity, but because the click handle blocks the 3'OH only a single base is attached to each oligo. After washing away the TdT, an additional index level is added using an identical procedure to the linkage of the first two oligonucleotides. Because the click reaction may not have 100% efficiency, further ligations can be blocked to avoid combinatorial indexes with 'skipped' levels. This is accomplished by adding a small molecule containing a complementary click handle to block any unreacted 3' groups.

In some embodiments, chemical ligation can be used in a splinted ligation strategy to reduce the amount of oligonucleotide needed for the reaction. Chemical ligation is compatible with the use of nonstandard nucleotides. The use of certain nonstandard nucleotides with higher hybridization specificities allows for shorter splint oligonucleotides. For example, modified nucleotides such as peptide nucleic acids (PNAs), locked nucleic acids (LNAs), or 2'-OMe bases can increase the melting temperature of an oligonucleotide without adding length.

FIG. 3 depicts an embodiment of a scheme to join a second index (index B) to a first index (index A) attached to a substrate by click chemistry ligation. As shown in FIG. 3, a first polynucleotide comprising a P5 sequence and an index A is joined to a bead via a biotin/streptavidin binding pair. For example, the bead is coated with biotin, and the first polynucleotide comprises a 5' end linked to a streptavidin; or the bead is coated with streptavidin, and the first polynucleotide comprises a 5' end linked to a biotin. The first polynucleotide comprises a 3' end with a propargyl moiety. A second polynucleotide comprises an index B and a 5' end with an azide moiety. A copper catalyzed click reaction results in joining of the first and second polynucleotides. Additional polynucleotides are added to the 3' end of the second polynucleotide by first treating the bead-linked polynucleotides with a TdT to add a single nucleotide comprising a propargyl moiety. An additional polynucleotide comprising a 5' end with an azide moiety can be used to further extend the bead-linked polynucleotides via an additional click reaction.

Some embodiments include extending a first polynucleotide, such as a bead-linked first polynucleotide, with a second polynucleotide by a chemical ligation reaction. In some embodiments, the first polynucleotide comprises a terminal 3'-modified deoxynucleotide (dNTP) comprising a 3' functional moiety capable of participating in a click chemistry reaction. In some embodiments, the second polynucleotide comprises a terminal 5' modified nucleotide comprising a compatible 5'-functional moiety capable of participating in a click chemistry reaction with the 3' functional moiety. In some embodiments, the 3' functional moiety and the 5' functional moiety are capable of reacting with one another to form a modified backbone linkage.

In some embodiments, extending the first polynucleotide with the second polynucleotide obtains a secondary indexed polynucleotide, and the method further comprises modifying the secondary indexed polynucleotide to obtain a modified polynucleotide comprising a terminal 3' modified nucleotide comprising a 3' functional moiety capable of participating in a click chemistry reaction. In some embodiments, the modifying comprises contacting the secondary indexed polynucleotide with a template independent polymerase. In some embodiments, the template independent polymerase is selected from a terminal deoxynucleotidyl transferase (TdT), PolyA polymerase, or CCA-adding RNA polymerase. In some embodiments, the template independent polymerase is TdT.

Some embodiments also include extending the modified polynucleotide with a third polynucleotide by a chemical ligation reaction, wherein the third polynucleotide comprises a terminal 5' modified nucleotide comprising a compatible 5' functional moiety capable of participating in a click chemistry reaction with the 3' functional moiety.

In some embodiments, the 3' functional moiety is selected from the group consisting of an azide, an alkynyl, an alkenyl, a thiol, and a nitrone. In some embodiments, the 5' functional moiety is different from and compatible with the 3' functional moiety and is selected from the group consisting of an azide, an alkynyl, an alkenyl, a thiol, and a nitrone In some embodiments, the 3' functional moiety and the 5' functional moiety are selected from the following pairs: (i) 3'-azido/5'-alkynyl; (ii) 3'-alkynyl/5' azido; (iii) 3'-thiol/5'-alkynyl; (iv) 3'-thiol/5'-alkenyl; (v) 3'-alkynyl/5'-thiol; (vi) 3'-alkenyl/5'-thiol; (vii) 3'-azido/5'-cyclooctynyl; (viii) 3'-cyclooctyne/5'-azido; (ix) 3'-nitrone/5'-cyclooctynyl; and (x) 3'-cyclooctynyl/5'-nitrone. In some embodiments, the 3' functional moiety is a 3'-azido and the 5' functional moiety is a 5'-alkynyl. In some embodiments, a TdT step can be avoided if orthogonal click reactions are used between the L1/L2 indexes and L2/L3 indexes.

In some embodiments, the click chemistry reaction comprises copper catalyzed azide-alkyne cycloaddition (CuAAC) to form a modified backbone linkage comprising a triazolyl.

Combinatorial indexing comprising polymerase extension

Some embodiments of the methods and compositions provided herein include preparation of indexed beads by combinatorial indexing in which a polynucleotide attached to a bead is extended by sequential addition of indexes by polymerase extension.

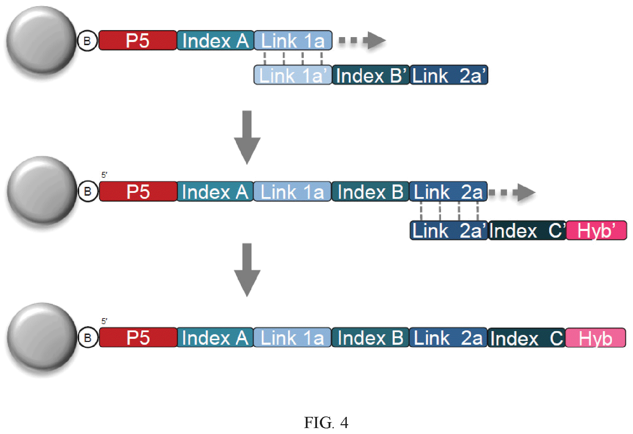

In some embodiments, an adaptor comprising oligonucleotide containing both a 3' region complementary to a 3' linker (L1A) region of a bead-bound first polynucleotide comprising a first index, and a reverse-complement of a second index sequence (FIG. 4). The adaptor oligonucleotide is hybridized to the bead-bound first polynucleotide, and a DNA polymerase and dNTPs extend the first polynucleotide, resulting in a covalently linked, bead-bound oligonucleotide containing the complement of the adaptor at its 3' end. Because the adaptor hybridizes directly to the first polynucleotide, half the number of bases are sufficient to achieve a similar specificity of binding, such as 8 bases vs. 16 bases for the current splint ligation workflow. Once polymerization is complete, the adaptor can be removed by denaturation using heat, base (NaOH), or treatment with a USER enzyme, for example if the adaptor comprises deoxyUracil residues in place of Thymine. Additional index levels can be added by hybridizing a second adaptor comprising a third index to the 3' end of the extended first polynucleotide and extending the extended first polynucleotide further with a polymerization reaction and the hybridized second adaptor. In some embodiments, to prevent the polymerase from using the 3' end of an adapter as a primer and generating undesired products, the 3' end of the adapter can be blocked, for example with a 3' 2' dideoxy nucleotide, C3 linker, or other standard blocking chemistry.

FIG. 4 depicts an embodiment of a scheme to add to a first index (index A) attached to a substrate, a second index (index B) and a third index (index C) by sequential polymerase extension reaction. As shown in FIG. 4, a first polynucleotide comprising a P5 sequence, an index A and a first linker (Link 1a) is joined to a bead via a biotin/streptavidin binding pair. For example, the bead is coated with biotin, and the first polynucleotide comprises a 5' end linked to a streptavidin; or the bead is coated with streptavidin, and the first polynucleotide comprises a 5' end linked to a biotin. A first adaptor comprising a region capable of hybridizing to the first linker, an index B', and a linker 2a' is hybridized to the first linker, and the first polynucleotide is extended in the presence of a polymerase to obtain an extended polynucleotide comprising indexes A and B. The first adaptor is removed. A second adaptor comprising a region capable of hybridizing to the linker 2a, an index C', and a capture probe' (Hyb') is hybridized to the linker 2a region of the extended polynucleotide comprising indexes A and B, and the extended polynucleotide comprising indexes A and B is further extended in the presence of a polymerase to obtain an extended polynucleotide comprising indexes A, B, and C, and a capture probe (Hyb).

Some embodiments include extending the first polynucleotide by polymerase extension. In some embodiments the first polynucleotide comprises a first linker. Some embodiments also include (i) obtaining a first adaptor comprising a region capable of hybridizing to the first linker and a region comprising the second index or complement of the second index; (ii) hybridizing the first adaptor to the first linker; and (iii) extending the first polynucleotide to obtain a secondary indexed polynucleotide. In some embodiments, the first adaptor comprises a non-extendable 3' end. In some embodiments, the non-extendable 3' end comprises a 3'2' dideoxy nucleotide, or a C3 linker, such as a 3-carbon spacer arm . Some embodiments also include removing the first adaptor from the secondary indexed polynucleotide. In some embodiments, the removing comprises denaturing the first adaptor by heat or base, or degrading the first adaptor by enzymic degradation. In some embodiments, the first linker has a length less than 10 9, 8, 7, 6, 5, 4, 3, or 2 consecutive nucleotides. In some embodiments, the first linker has a length less than 5 consecutive nucleotides.

In some embodiments, obtaining a population of tertiary indexed beads comprises extending the second polynucleotide by polymerase extension. In some embodiments, the first adaptor comprises a complement of a second linker, such that the secondary indexed polynucleotide comprises the second linker. Some embodiments also include (i) obtaining a second adaptor comprising a region capable of hybridizing to the second linker and a region comprising the third index or complement of the third index; (ii) hybridizing the second adaptor to the second linker; and (iii) extending the

secondary indexed polynucleotide to obtain a tertiary indexed polynucleotide. In some embodiments, the second adaptor comprises a non-extendable 3' end. In some embodiments, the non-extendable 3' end comprises a 3'2' dideoxy nucleotide, or a C3 linker. Some embodiments also include removing the second adaptor from the tertiary indexed polynucleotide. In some embodiments, the removing comprises denaturing the first adaptor by heat or base, or degrading the first adaptor by enzymic degradation. In some embodiments, the second linker has a length less than 10, 9, 8, 7, 6, 5, 4, 3, or 2 consecutive nucleotides. In some embodiments, the second linker has a length less than 5 consecutive nucleotides.

Combinatorial indexing comprising ligation of double-stranded fragments

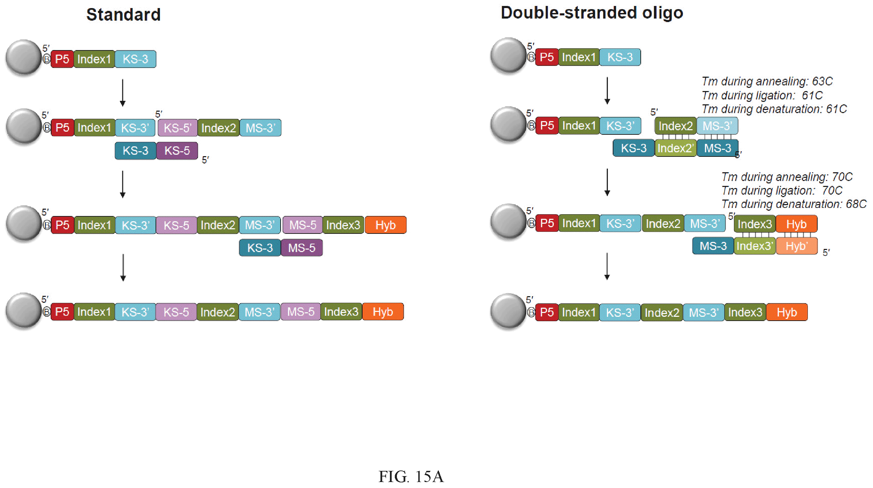

Some embodiments of the methods and compositions provided herein include preparation of indexed beads by combinatorial indexing in which a polynucleotide attached to a bead is extended by sequential addition of indexes by ligation of an adaptor comprising a second polynucleotide and a double-stranded region. In some embodiments, the use of a partially double-stranded adaptor means that a single linker sequence can be used to ligate an additional index to a bead-linked oligonucleotide, thereby reducing the length of a linker sequence in a combinatorially indexed oligonucleotide compared to some other methods. For example, FIG. 15A (left panel) shows an example embodiment for a standard splint ligation in which the splint contains two linker sequences "KS-3" and "KS-5" which anneal to a "KS-3'" sequence in a bead-linked oligonucleotide and to a "KS-5'" sequence in an adaptor sequence containing Index2, respectively. In contrast, the example embodiment of double stranded splint ligation shown in FIG. 15A (right panel) shows a double-stranded splint which contains a single linker sequence "KS-3" sufficient for ligating an adaptor sequence containing an index to a bead-linked oligonucleotide.

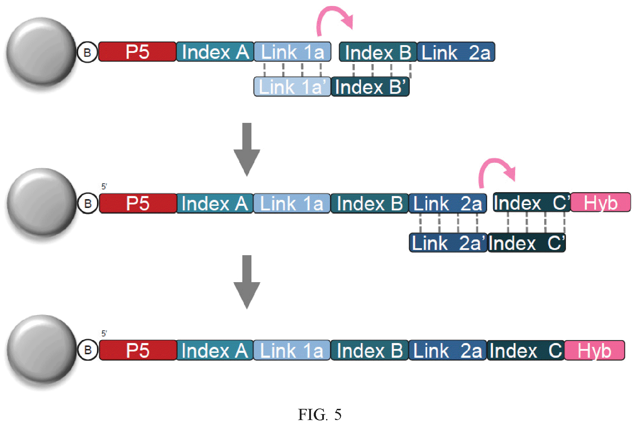

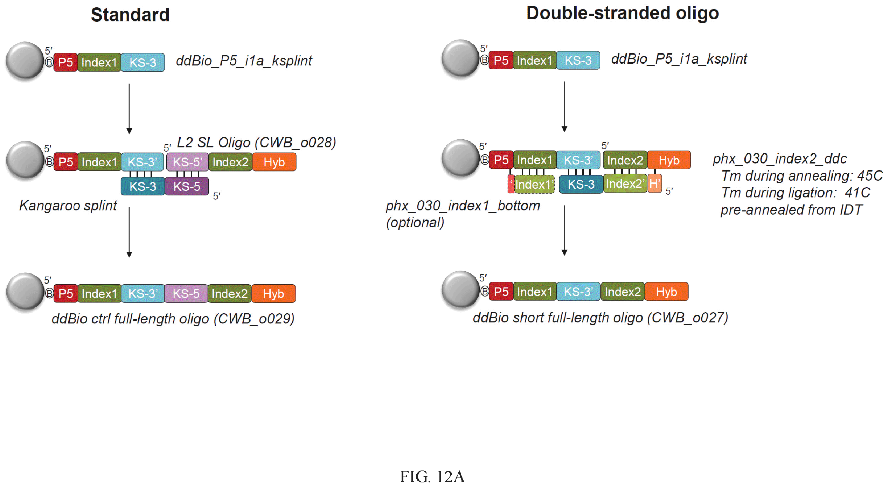

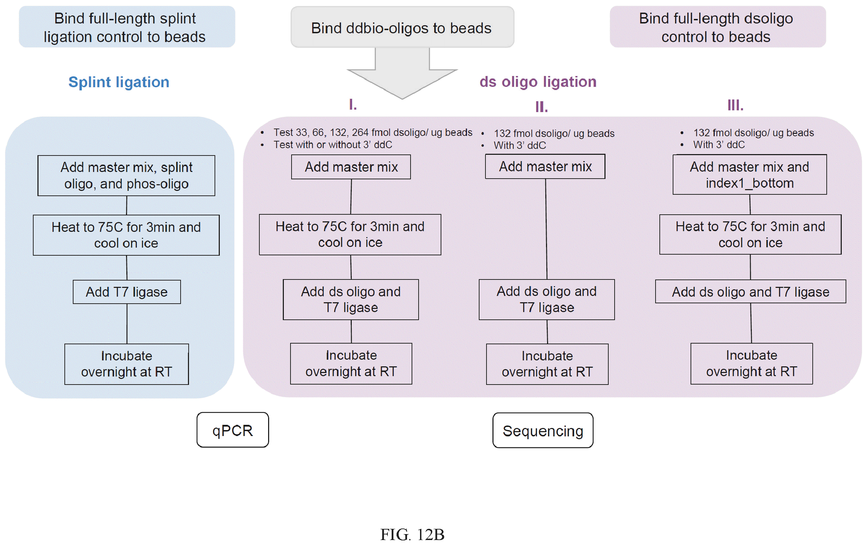

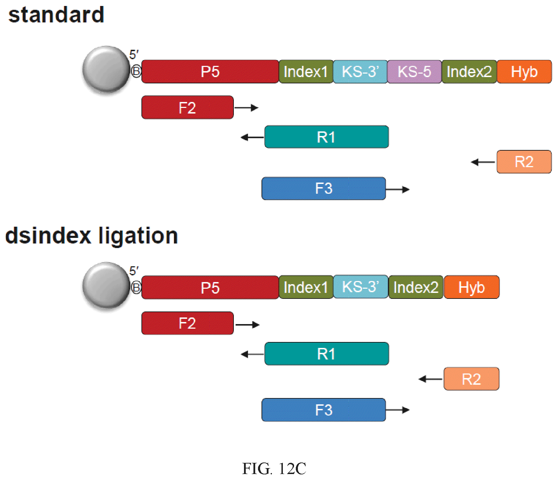

Some embodiments include the use of double stranded adapters comprising index oligonucleotides with complementary overhangs extending from the 3' end of both strands of the duplex (*see e.g.*, FIG. 5, FIG. 15A, and FIG. 19A). Some such embodiments reduce by up to 50% the number of invariant bases in the Bead code as only a single region of complementary bases is sufficient for assembly. Beginning with a bead-bound first polynucleotide comprising a first index, an adaptor comprising a double-stranded fragment with a 3' overhang complementary to the 3' end of the first polynucleotide is added to the beads and allowed to hybridize. The 'top' fragment, containing the index sequence and a 3' hybridization sequence for the next index level, is then linked to the first polynucleotide by enzymatic or chemical ligation. Once ligation is complete, the bottom strand of the double-stranded index portion of the adaptor can be removed by denaturation, and additional index levels can be added by the same procedure. In some embodiments, an additional single-stranded oligonucleotide may be present during the ligation (see e.g., FIG. 12, right panel 'index1' oligo'). The additional oligonucleotide can hybridize to an index sequence of the bead-bound polynucleotide. In some such embodiments, the additional oligonucleotide may further stabilize the ligation complex and promote ligation.

FIG. 5 depicts an embodiment of a scheme to add to a first index (index A) attached to a substrate, a second index (index B) and a third index (index C) using an adaptor comprising a double-stranded region and single-stranded overhang. As shown in FIG. 5, a first polynucleotide comprising a P5 sequence, an index A and a first linker (Link 1a) is joined to a bead via a biotin/streptavidin binding pair. For example, the bead is coated with biotin, and the first polynucleotide comprises a 5' end linked to a streptavidin; or the bead is coated with streptavidin, and the first polynucleotide comprises a 5' end linked to a biotin. A first adaptor comprising a single stranded 5' overhang comprising a region capable of hybridizing to the first linker, a double stranded region comprising an index B, and a region comprising a second linker (link 2a) is hybridized to the first linker. The first adaptor is covalently joined to the first polynucleotide via the linker 1a and index B regions of the first polynucleotide and adaptor, respectively by ligation with a ligase, or by chemical ligation to obtain an extended polynucleotide comprising indexes A and B. The strand of the first adaptor not covalently joined to the first polynucleotide is removed. A second adaptor comprising a single stranded 5' overhang comprising a region capable of hybridizing to the second linker, a double stranded region comprising an index C and a capture probe (Hyb) is hybridized to the second linker. The second adaptor is covalently joined to the extended first polynucleotide via the linker 1a and index B regions of the first polynucleotide and adaptor, respectively by ligation with a ligase, or by chemical ligation to obtain an extended polynucleotide comprising indexes A, B, and C and a capture probe (Hyb). The strand of the second adaptor not covalently joined to the extended polynucleotide comprising indexes A, B, and C and a capture probe (Hyb is removed.

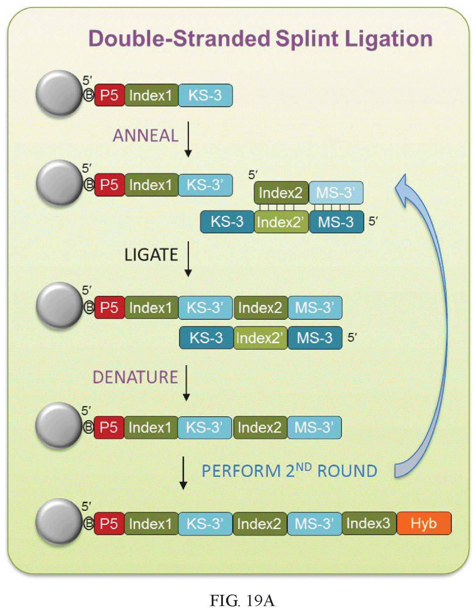

FIG. 19A depicts an additional example embodiment of combinatorial indexing in which a polynucleotide attached to a bead is extended by sequential addition of indexes by ligation of an adaptor comprising a second polynucleotide and a double-stranded region. As shown in FIG. 19A, an oligonucleotide comprising a P5 sequence, an index1, and a KS-3' sequence is linked to a bead via biotin (B) and streptavidin (SA). A partially double-stranded adapter in which one strand comprises a KS-3 sequence, an index2' and an MS-3 sequence, and the other strand comprises an index2 sequence and an MS-3' sequence, is annealed to the bead-linked oligonucleotide via the KS-3 and KS-3' sequences. Adapter sequences are ligated to bead-linked sequences to form an extended bead-linked oligonucleotide hybridized to a strand of the adapter. The strand of the adapter is removed by denaturation. A second round of indexing is performed.

Some embodiments include extending the first polynucleotide with the second polynucleotide by ligation. Some

embodiments also include (i) obtaining a double stranded first adaptor comprising the second polynucleotide and a 3' single stranded overhang capable of hybridizing to a first linker of the first polynucleotide; (ii) hybridizing the first adaptor to the first linker; and (iii) ligating the first polynucleotide to the second polynucleotide to obtain a secondary indexed polynucleotide. Some embodiments also include extending the second polynucleotide with a third polynucleotide by ligation. Some embodiments also include (i) obtaining a double stranded second adaptor comprising the third polynucleotide and a 3' single stranded overhang capable of hybridizing to a second linker of the second polynucleotide; (ii) hybridizing the second adaptor to the second linker; and (iii) ligating the second polynucleotide to the third polynucleotide to obtain a tertiary indexed polynucleotide. In some embodiments, the ligation comprises use of a ligase. In some embodiments, the ligation comprises a chemical ligation reaction, such as a click chemistry reaction disclosed herein.

Combinatorial indexing comprising splint ligation

Some embodiments of the methods and compositions provided herein include preparation of indexed beads by combinatorial indexing in which a polynucleotide attached to a bead is extended by sequential addition of indexes by splint ligation in which the splint comprises nucleotides having increased hybridization specificity. In some embodiments, the splint comprises nucleotides that have stronger binding with a complementary sequence, for example a nucleotide sequence comprising nucleotides that result in an increased Tm for the nucleotide sequence. In some embodiments, the splint comprises one or more modified nucleotides, or nucleotide analogs. In some embodiments, the splint includes a locked nucleic acid (LNA). In some embodiments, the splint comprises one or more inosine nucleotides.

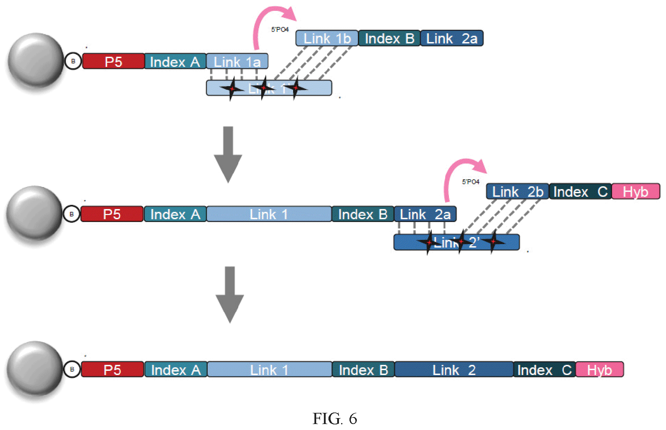

Some embodiments include the use of splinted ligation to link successive levels of an index with a reduced number of bases in the splint comprising chemically modified bases which increase the strength of hybridization between the splint and the index/capture oligonucleotides (FIG. 6). In their current form, the splint sequences are 8 nucleotides long with a midpoint of denaturation (Tm) close to room temperature (~25°C). This ensures that the splint can bind both the capture oligonucleotide and the index oligonucleotide during the roomtemperature ligation reaction.

FIG. 6 depicts an embodiment of a scheme to add to a first index (index A) attached to a substrate, a second index (index B) and a third index (index C) by sequential splinted ligation reactions with short linker-splints. As shown in FIG. 6, a first polynucleotide comprising a P5 sequence, an index A and a first linker (Link 1a) is joined to a bead via a biotin/streptavidin binding pair. For example, the bead is coated with biotin, and the first polynucleotide comprises a 5' end linked to a streptavidin; or the bead is coated with streptavidin, and the first polynucleotide comprises a 5' end linked to a biotin. A second polynucleotide comprises a second linker (Link1b), an index B, and a third linker (Link 2a). A single-stranded first adaptor, such as a splint, comprising a region capable of hybridizing to the first linker and a region capable of hybridizing to the second linker is hybridized to both the first linker of the first polynucleotide, and to the second linker of the second polynucleotide. The first polynucleotide is covalently joined to the second polynucleotide by ligation, such as by use of a ligase to obtain an extended polynucleotide comprising indexes A and B and a third linker (Link 2a). The first adaptor is removed. A third polynucleotide comprises a fourth linker (Link 2b), an index C, and a capture probe (Hyb). A single-stranded second adaptor, such as a splint, comprising a region capable of hybridizing to the third linker and a region capable of hybridizing to the fourth linker is hybridized to both the third linker of the extended polynucleotide and to the fourth linker of the third polynucleotide. The third polynucleotide is covalently joined to the extended polynucleotide by ligation, such as by use of a ligase to obtain an extended polynucleotide comprising indexes A, B, and C, and the capture probe. The second adaptor is removed.

Some embodiments include extending the first polynucleotide by ligation, wherein the first polynucleotide comprises a first linker, and the second polynucleotide comprises a second linker. Some embodiments also include (i) obtaining a first adaptor comprising a region capable of hybridizing to the first linker and a region capable of hybridizing to the second linker; (ii) hybridizing the first adaptor to the first linker; (iii) hybridizing the second oligonucleotide to the region capable of hybridizing to the second linker; and (iv) ligating the first polynucleotide to the second polynucleotide to obtain a secondary indexed polynucleotide. In some embodiments, the first linker and/or the second linker has a length less than 10, 9, 8, 7, 6, 5, 4, 3, or 2 consecutive nucleotides. In some embodiments, the first linker and/or the second linker has a length less than 5 consecutive nucleotides. In some embodiments, the first linker and/or the second linker is modified to have an increased Tm compared to an oligonucleotide having the same length as the first adaptor. In some embodiments, the first linker and/or the second linker comprises an increased G/C content compared to the oligonucleotide having the same length, or comprises modified nucleotides. Some embodiments also include removing the first adaptor from the secondary indexed polynucleotide. In some embodiments, the removing comprises denaturing the first adaptor by heat or base, or degrading the first adaptor by enzymic degradation.

In some embodiments, obtaining a population of tertiary indexed beads comprises extending the second polynucleotide by ligation, wherein the second oligonucleotide comprises a third linker, such that the secondary indexed polynucleotide comprises the third linker, and wherein the third polynucleotide comprises a fourth linker. Some embodiments also include (i) obtaining a second adaptor comprising a region capable of hybridizing to the third linker and a region capable of hybridizing to the fourth linker; (ii) hybridizing the second adaptor to the third linker; (iii) hybridizing the third

polynucleotide to the second adaptor via the region capable of hybridizing to the fourth index; and (iv) ligating the secondary indexed polynucleotide to the third polynucleotide to obtain a tertiary indexed polynucleotide. In some embodiments, the third linker and/or the fourth linker has a length less than 9, 8, 7, 6, 5, 4, 3, or 2 consecutive nucleotides. In some embodiments, the third linker and/or the fourth linker has a length less than 5 consecutive nucleotides. In some embodiments, the third linker and/or the fourth linker is modified to have an increased Tm compared to an oligonucleotide having the same length as the second adaptor. In some embodiments, the third linker and/or the fourth linker comprises an increased G/C content compared to the oligonucleotide having the same length, or comprises modified nucleotides. Some embodiments also include removing the second adaptor from the tertiary indexed polynucleotide. In some embodiments, the removing comprises denaturing the first adaptor by heat or base, or degrading the first adaptor by enzymic degradation.

Sequencing and analysis of target nucleic acids

Some embodiments include sequencing and/or analysis of target nucleic acids. Certain methods and compositions useful with embodiments provided herein are disclosed in U.S. 2021/0087613 which is incorporated by reference in its entirety. Some embodiments include decoding the locations of polynucleotides in an array according to methods provided herein; hybridizing target nucleic acid to capture probes; extending the capture probes; and detecting the extension of the capture probes hybridized to the target nucleic acid at a location on the array. In some embodiments, the locations of the polynucleotides on an array can be decoded before hybridizing target nucleic acid to the polynucleotides. In some embodiments, the locations of the polynucleotides on an array can be decoded after detecting the extension of the capture probes hybridized to the target nucleic acid. In some such embodiments, each polynucleotide can be associated with a capture probe through a common element. For example, a polynucleotide and a capture probe can each be bound to the same microfeature, such as a bead. In more such embodiments, each polynucleotide can include the capture probe.

Some embodiments include single base extension (SBE) of capture probes. In some embodiments, SBE can be used for detection of an allele, mutations or other features in target nucleic acids. Briefly, SBE utilizes a capture probe that hybridizes to a target genome fragment at a location that is proximal or adjacent to a detection position, the detection position being indicative of a particular locus. A polymerase can be used to extend the 3' end of the capture probe with a nucleotide analog labeled with a detection label. Based on the fidelity of the enzyme, a nucleotide is only incorporated into the capture probe if it is complementary to the detection position in the target nucleic acid. If desired, the nucleotide can be derivatized such that no further extensions can occur using a blocking group, including reversible blocking groups, and thus only a single nucleotide is added. The presence of the labeled nucleotide in the extended capture probe can be detected for example, at a particular location in an array and the added nucleotide identified to determine the identity of the locus or allele. SBE can be carried out under known conditions such as those described in U.S. Pat. Nos. 9,441,267 and 9,045,796 each of which is incorporated by reference in its entirety.

Some embodiments include allele specific primer extension (ASPE). In some embodiments ASPE can include extension of capture probes that differ in nucleotide composition at their 3' end. An ASPE method can be performed using a nucleoside or nucleotide containing a cleavable linker, so that a label can be removed after a probe is detected. This allows further use of the probes or verification that the signal detected was due to the label that has now been removed. Briefly, ASPE can be carried out by hybridizing a target nucleic acid to a capture probe having a 3' sequence portion that is complementary to a detection position and a 5' portion that is complementary to a sequence that is adjacent to the detection position. Template directed modification of the 3' portion of the capture probe, for example, by addition of a labeled nucleotide by a polymerase yields a labeled extension product, but only if the template includes the target sequence. The presence of such a labeled primer-extension product can then be detected, for example, based on its location in an array to indicate the presence of a particular allele. In some embodiments, ASPE can be carried out with multiple capture probes that have similar 5' ends such that they anneal adjacent to the same detection position in a target nucleic acid but different 3' ends, such that only capture probes having a 3' end that complements the detection position are modified by a polymerase. A capture probe having a 3' terminal base that is complementary to a particular detection position is referred to as a perfect match (PM) probe for the position, whereas capture probes that have a 3' terminal mismatch base and are not capable of being extended in an ASPE reaction are mismatch (MM) probes for the position. The presence of the labeled nucleotide in the PM probe can be detected and the 3' sequence of the capture probe determined to identify a particular allele at the detection position.

Kits and systems

Some embodiments provided herein include kits and systems. Some such embodiments can include reagents to perform certain methods provided herein including, beads, multiwell plates, enzymes, such as ligases and polymerases, polynucleotides, binding-pairs such as biotin and streptavidin or derivatives thereof, and click chemistry reagents.

EXAMPLES

Example 1-Bead code synthesis by click chemical ligation

A plurality of beads are coated with streptavidin and are distributed in the wells of a first 96 well plate. A different first polynucleotide is distributed into each well. Each first polynucleotide comprises a 5' end linked to a biotin, such that the first polynucleotide is joined to a bead via the biotin/streptavidin binding pair. The first polynucleotide comprises a P5 sequence, an index A, and a 3' end with a propargyl moiety. The first polynucleotide in each well has a different index A from a first polynucleotide in a different well. The beads are pooled and distributed into the wells of a second 96 well plate.

A second polynucleotide is added to each well of the second 96 well plate. The second polynucleotide comprises an index B and a 5' end with an azide moiety. The index B is different for each well. A copper catalyzed click reaction is performed in each well and results in joining of the first and second polynucleotides. The bead-linked polynucleotides are treated with a TdT to add a single nucleotide comprising a propargyl moiety. The beads are pooled, and distributed into the wells of a third 96 well plate.

Additional polynucleotides are added to the 3' end of the second polynucleotide. A third polynucleotide comprising a 5' end with an azide moiety and an index C can be used to further extend the bead-linked polynucleotides via an additional click reaction. The index C is different for each well.

Example 2-Combinatorial indexing comprising polymerase extension

A plurality of beads are coated with streptavidin, and are distributed in the wells of a first 96 well plate. A different first polynucleotide is distributed into each well. Each first polynucleotide comprises a 5' end linked to a biotin, such that the first polynucleotide is joined to a bead via the biotin/streptavidin binding pair. The first polynucleotide comprises a P5 sequence, an index A, and an 8 nucleotide first linker. The first polynucleotide in each well has a different index A from a first polynucleotide in a different well. The beads are pooled and distributed into the wells of a second 96 well plate.

A first adaptor is added to each well of the second 96 well plate. The first adaptor comprises a region capable of hybridizing to the first linker, an index B' sequence, and a linker 2a'. The index B' sequence is different for each well. The first adaptor is hybridized to the first linker, and the first polynucleotide is extended in the presence of a polymerase to obtain an extended polynucleotide comprising indexes A and B. The first adaptor is removed from the beads. The beads are pooled and distributed into the wells of a third 96 well plate.

A second adaptor is added to each well of the third 96 well plate. The second adaptor comprises a region capable of hybridizing to the linker 2a, an index C', and a capture probe' (Hyb'). In some embodiments, the capture probe is useful for a specific application where a universal transposome is attached to the 3' end of the bead-linked oligo, and can be is unrelated to indexing (i.e. this could be any sequence depending on the application). The index C' sequence is different for each well. The second adaptor is hybridized to the linker 2a region of the extended polynucleotide comprising indexes A and B, and the extended polynucleotide comprising indexes A and B is further extended in the presence of a polymerase to obtain an extended polynucleotide comprising indexes A, B, and C, and a capture probe (Hyb). The second adaptor is removed from the beads.

Example 3-Combinatorial indexing comprising ligation of double-stranded fragments

A plurality of beads are coated with streptavidin and are distributed in the wells of a first 96 well plate. A different first polynucleotide is distributed into each well. Each first polynucleotide comprises a 5' end linked to a biotin, such that the first polynucleotide is joined to a bead via the biotin/streptavidin binding pair. The first polynucleotide comprises a P5 sequence, an index A, and an 8 nucleotide first linker. The first polynucleotide in each well has a different index A from a first polynucleotide in a different well. The beads are pooled and distributed into the wells of a second 96 well plate.

A first adaptor is added to each well of the second 96 well plate. The first adaptor comprises a single stranded 5' overhang comprising a region capable of hybridizing to the first linker, a double stranded region comprising an index B, and a region comprising a second linker (link 2a). The index B is different for each well. The first adaptor is hybridized to the first linker. The first adaptor is covalently joined to the first polynucleotide via the linker 1a and index B regions of the first polynucleotide and adaptor, respectively by ligation with a ligase, or by chemical ligation to obtain an extended polynucleotide comprising indexes A and B. The strand of the first adaptor not covalently joined to the first polynucleotide is removed. The beads are pooled and distributed into the wells of a third 96 well plate.

A second adaptor is added to each well of the third 96 well plate. The second adaptor comprises a single stranded 5' overhang comprising a region capable of hybridizing to the second linker, a double stranded region comprising an index C and a capture probe (Hyb). The second adaptor is hybridized to the second linker. The second adaptor is covalently joined to the extended first polynucleotide via the linker 1a and index B regions of the first polynucleotide and adaptor, respectively by ligation with a ligase, or by chemical ligation to obtain an extended polynucleotide comprising indexes A, B,

and C and a capture probe (Hyb). The strand of the second adaptor not covalently joined to the extended polynucleotide comprising indexes A, B, and C and a capture probe (Hyb) is removed from the beads.

Example 4-Combinatorial indexing comprising splint ligation

A plurality of beads are coated with streptavidin and are distributed in the wells of a first 96 well plate. A different first polynucleotide is distributed into each well. Each first polynucleotide comprises a 5' end linked to a biotin, such that the first polynucleotide is joined to a bead via the biotin/streptavidin binding pair. The first polynucleotide comprises a P5 sequence, an index A, and a first linker. The first polynucleotide in each well has a different index A from a first polynucleotide in a different well. The beads are pooled and distributed into the wells of a second 96 well plate.

A second polynucleotide is added to each well of the second 96 well plate. The second polynucleotide comprises a second linker (Link1b), an index B, and a third linker (Link 2a). The index B is different for each well. A single-stranded first adaptor, such as a splint, comprising a region capable of hybridizing to the first linker and a region capable of hybridizing to the second linker is added to each well. The first adaptor is hybridized to both the first linker of the first polynucleotide, and to the second linker of the second polynucleotide. The first polynucleotide is covalently joined to the second polynucleotide by ligation, such as by use of a ligase to obtain an extended polynucleotide comprising indexes A and B and a third linker (Link 2a). The first adaptor is removed. The beads are pooled and distributed into the wells of a third 96 well plate.

A third polynucleotide is added to each well of the third 96 well plate. The third polynucleotide comprises a fourth linker (Link 2b), an index C, and a capture probe (Hyb). The index B is different for each well. A single-stranded second adaptor, such as a splint, comprising a region capable of hybridizing to the third linker and a region capable of hybridizing to the fourth linker is added to each well. The second adaptor is hybridized to both the third linker of the extended polynucleotide and to the fourth linker of the third polynucleotide. The third polynucleotide is covalently joined to the extended polynucleotide by ligation, such as by use of a ligase to obtain an extended polynucleotide comprising indexes A, B, and C, and the capture probe. The second adaptor is removed from the beads.

Example 5-Combinatorial indexing comprising polymerase extension

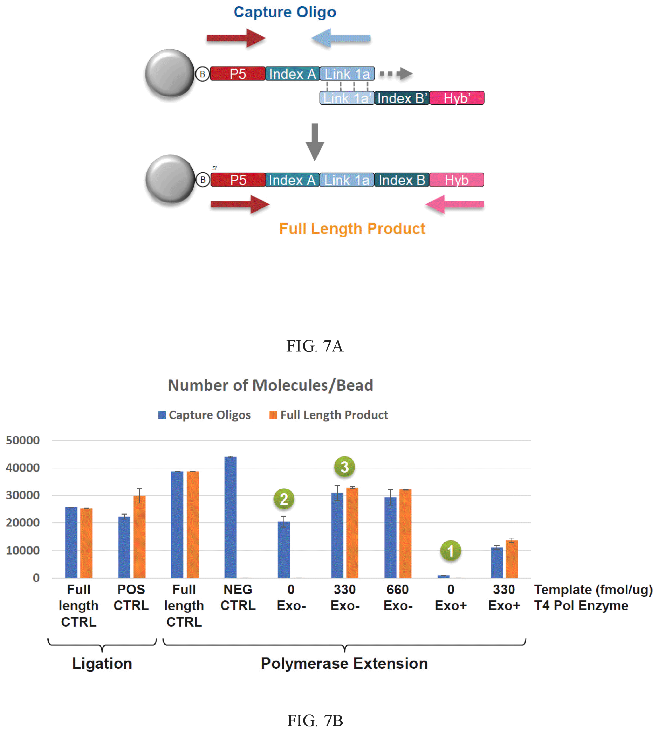

Combinatorial indexing comprising polymerase extension (see e.g., FIG. 4) was compared to combinatorial indexing comprising splint ligation (see e.g., FIG. 2). A 2-level indexing protocol by polymerase extension was performed using beads with a capture oligonucleotide (P5-index A-Link 1a), and an extension template (Link 1a'-index B'-Hyb'). Polymerase extension was performed with different amounts of template, and T4 polymerases with and without exonuclease activity. The number of capture probes and the number of full-length products were measured using quantitative PCR (qPCR) with primers at positions shown in FIG. 7A. As shown in FIG. 7B, (1) T4 polymerase (Exo+) degraded capture probes and extension templates; (2) T4 polymerase (Exo-) exhibited reduced degradation of capture oligonucleotides; (3) 50X excess extension template (330 fmol/ug) over capture oligo, was sufficient for full-length extension using T4 Polymerase (Exo-).

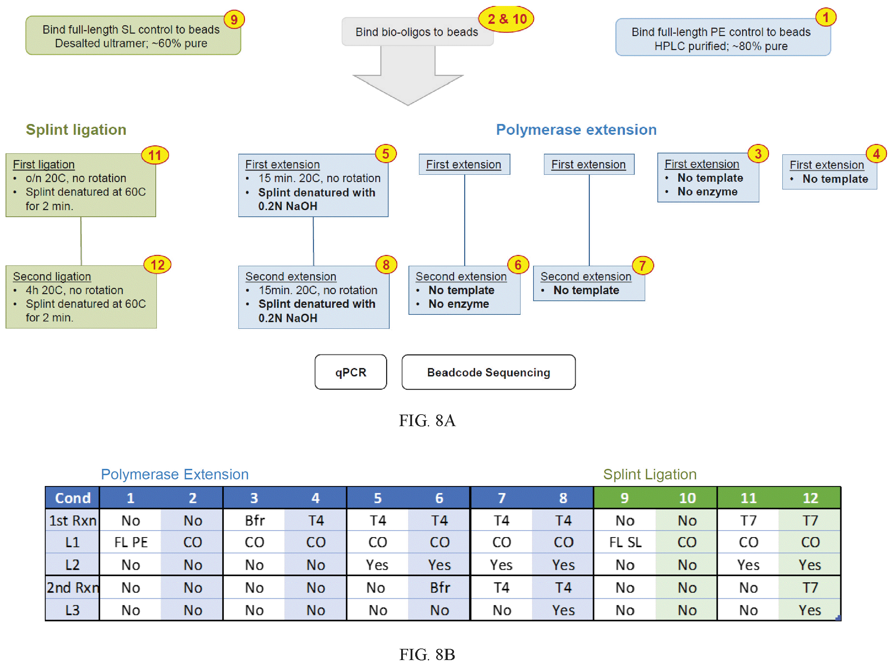

FIG. 8A and FIG. 8B summarize various conditions tested in a 3-level indexing protocol. Volumes were 25 μl/condition in duplicates. Polymerase extension was performed for 15 minutes at 20°C (no rotation) and with NaOH extension oligo denaturation. Splint ligation was performed overnight and for 4 hours (no rotation) and with 60°C splint denaturation. Readouts included a quantitative PCR (qPCR) assay, and direct beadcode sequencing. For qPCR, a chemically synthesized full-length control oligo (sequence identical to the corresponding 3-level PE or SL oligo) was used to create a standard curve for quantitation. Concentrations of splint ligation (SL) 2nd-level and 3rd-level amplicons were adjusted for size difference.

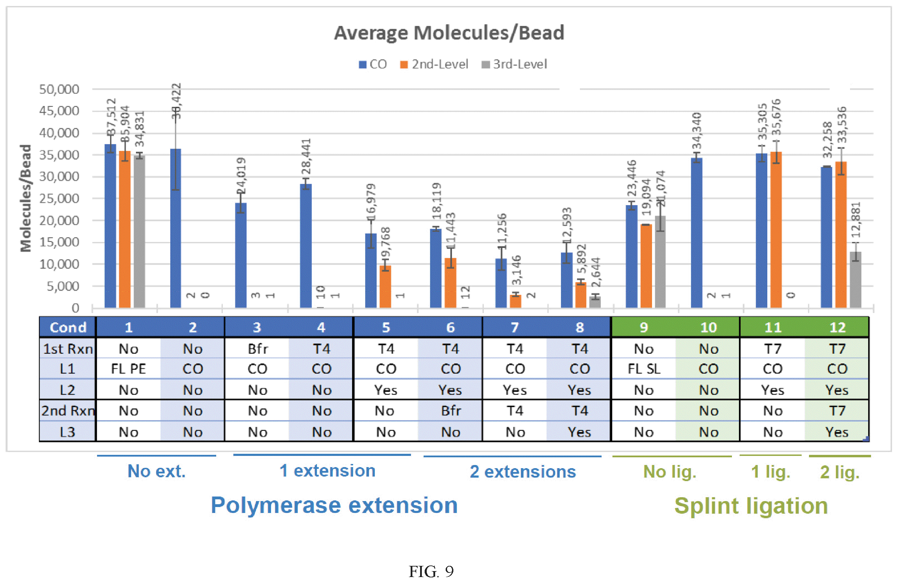

FIG. 9 summarizes qPCR results and shows that PE (8) gives fewer full-length oligos than SL (12). Efficiency of synthesis was lower for PE vs SL, for example, PE: 20% of oligos are full-length, and SL: 40% of oligos are full-length which may have been due to the second ligation being much less efficient than 1st ligation (12), and/or to 20°C incubation without rotation. Samples that underwent PE showed lower capture oligo (CO) molecules/bead (3-8) compared to full length (FL) PE (1) and SL (10-12). Each extension reduced CO molecules/bead (3-5 vs 6-8). Some loss was due to NaOH wash stripping Bio-oligos off beads. Some loss may have been due to T4 polymerase chewing back the oligos as in (6) vs (7&8), and in (3) vs (5), but this was not observed in (3) vs (4).

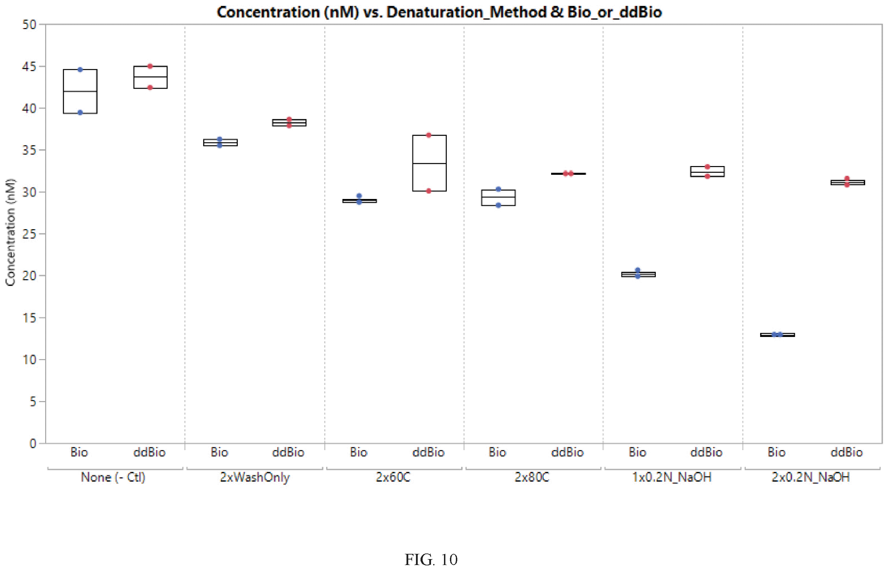

Effects of 0.2N NaOH in a denaturation step were tested. Beads with 6.6 fmol 1st level oligo (B1 index) bound to beads via either 5' biotin or 5' ddBiotin were used with no extension / ligation in 5 different denaturation treatments and a control: none (-control); no denaturation, just wash step (2 washes per level, 4 total); 2x 60°C Heat denature + washes; 2x 80°C Heat denature + washes; 1x 0.2N NaOH denature + wash; and 2x 0.2N NaOH denature + washes. Remaining oligo was measured by qPCR w/ 1st level primers. Results are summarized in FIG. 10 which shows that 0.2N NaOH denaturation caused biotin-specific CO loss.

A direct beadcode sequencing analysis was performed for 3-level indexing. Briefly, this assay involves amplifying the synthesized beadcode oligos off of the beads by PCR, using a P5 forward primer and a reverse primer targeting the Hyb

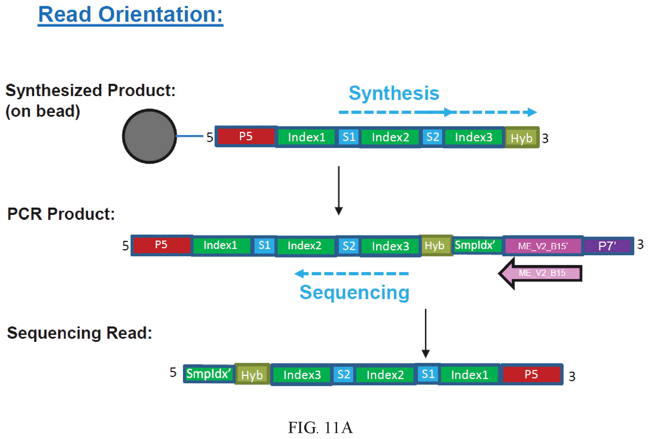

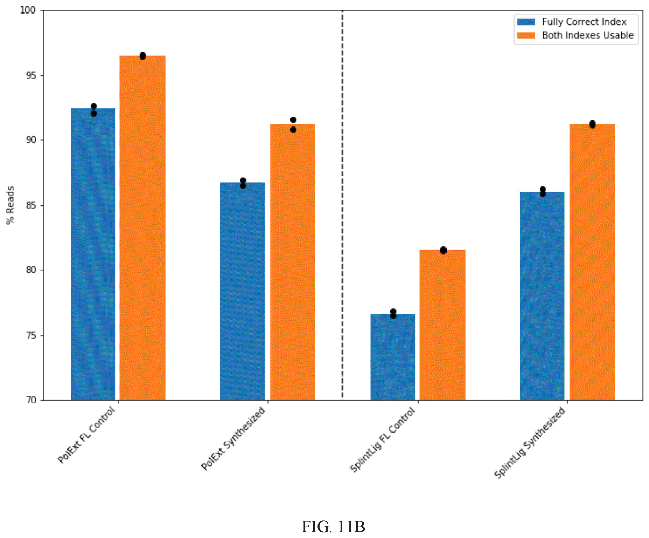

region. The reverse primer introduces a sample index, a binding region for the ME_V2_B15 sequencing primer, and a P7' sequence to enable clustering. FIG. 11A outlines a read orientation for the analysis. The analysis includes trimming and pairwise alignment of each read against an expected sequence with error calling including identification of deletions and putative 'SNP's which indicate base change errors in a sequence. Fully correct indexes included no errors (SNPs, indels) across an index region, for example in a 3-level index: index 1, index 2, and index 3. All usable indexes included those indexes which could be decoded correctly with an index error correction implementation. FIG. 11B and FIG. 11C show levels for fully correct sequences, usable indexes, and per base error rates. Splint ligation was more efficient than polymerase extension, while both had similar levels of errors.

From the foregoing studies, 3-level polymerase extension produced fewer full-length molecules than splint ligation. Addition of 0.2N NaOH denaturation and T4 polymerase exonuclease activity likely contributed to reduction in total capture oligos. Polymerase extension appeared to be less efficient than splint ligation in this experiment. 0.2N NaOH denaturation led to loss of biotinylated oligos from the bead, though this can be mitigated by replacing the single 5' biotin with a 5' dual desthiobiotin. Synthesized polymerase extension oligos had similar levels of errors compared to the splint ligation method.

Example 6-2-level indexing with double-stranded splint ligation

2-level indexing comprising double-stranded splint ligation was compared to 2-level indexing comprising splint ligation (standard). An overview of standard combinatorial indexing by splint ligation, and double-stranded splint ligation is shown in FIG. 12A. Compared to standard splint ligation, in the double-stranded method, the 5' half of the splint may be complementary to the second level index, reducing the common sequence length between indices in half. An overview of protocols and conditions tested are depicted in FIG. 12B.

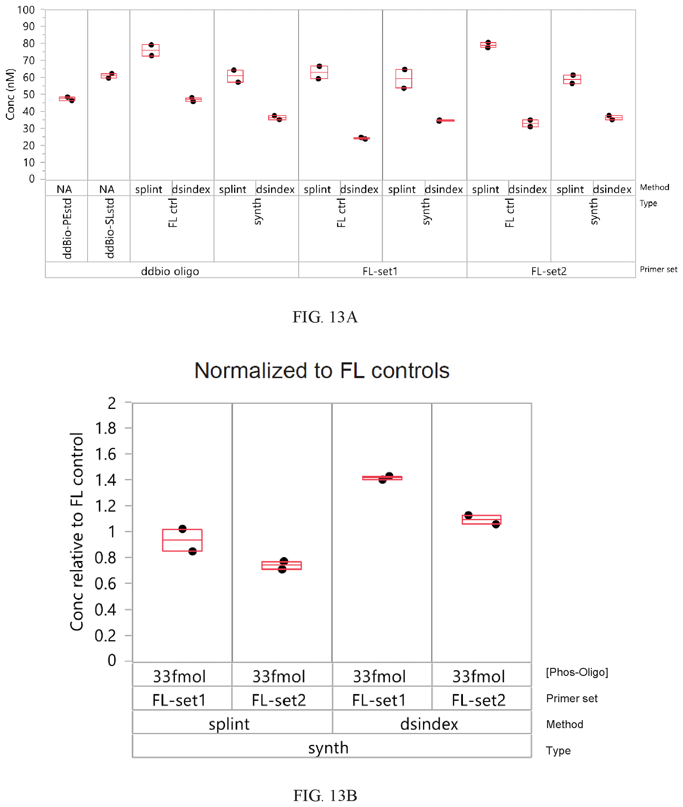

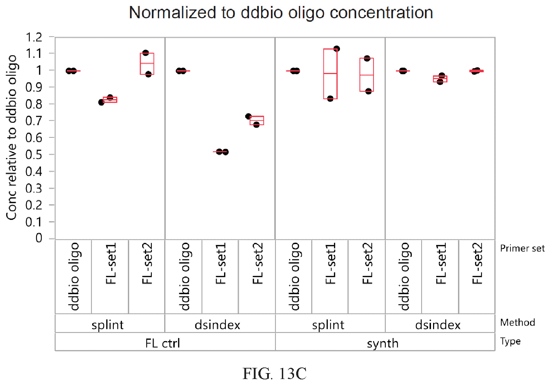

A qPCR assay was performed and full-length splint ligation and double-stranded ligation oligos were used to generate standard curves. Primer positions are shown in FIG. 12C for combinatorial indexing comprising splint ligation (standard), and combinatorial indexing comprising double stranded splint ligation (dsindex ligation) in which products were determined using primer combinations including: F2 and R2 or F3 and R2. Concentrations were determined using the appropriate standard curve for each sample. FIG 13A depicts the results of the qPCR, and FIG. 13B and FIG. 13C depicts results for normalized qPCR in which synthesized ds index ligation oligos were similar to splint ligation oligos. After normalizing for differential amplification between the SL and DS full-length control oligos, DS-ligated and splint-ligated oligos showed similar performance.

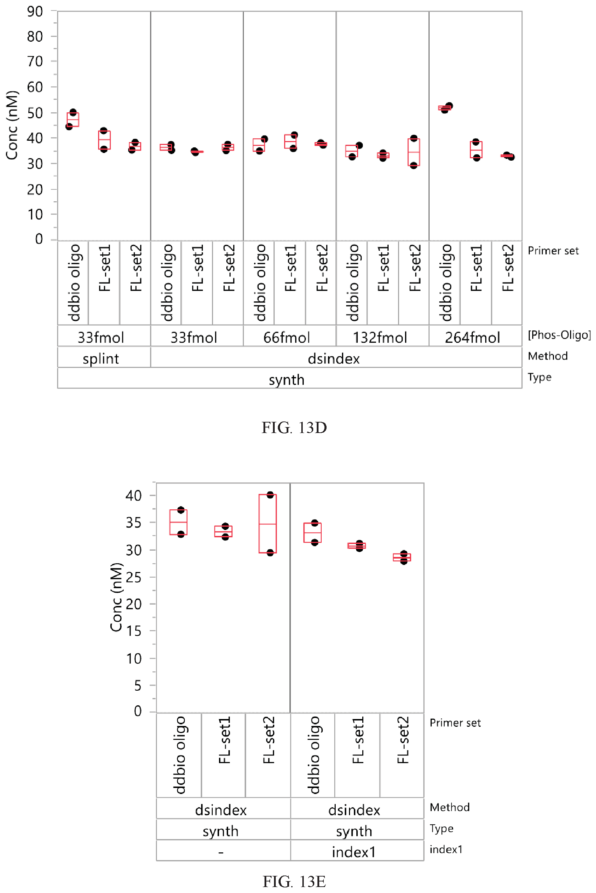

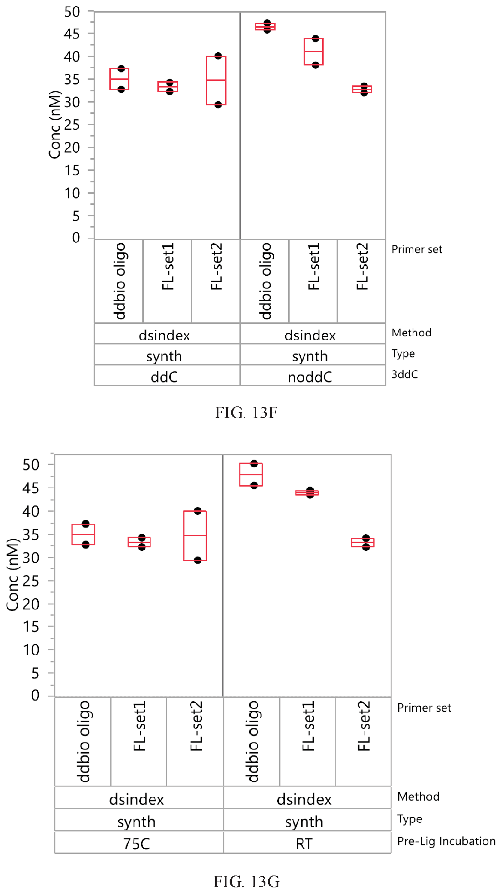

Double-stranded splint ligation was performed with increasing amounts of the ds index oligo, and the products measured with qPCR. As depicted in FIG. 13D, there was no substantial increase in the amount of product. Double-stranded splint ligation was performed in the presence of an oligo complementary to index 1, and the products measured with qPCR. As depicted in FIG. 13E, there was no substantial increase in the amount of product where double-stranded splint ligation was performed in the presence of an oligo complementary to index 1. Double-stranded splint ligation was performed with an oligo having a 3'ddC, and the products measured with qPCR. As depicted in FIG. 13F, there was no substantial increase in the amount of product where double-stranded splint ligation was performed with an oligo having a 3'ddC. Double-stranded splint ligation was performed including pre-ligation incubation at either room temperature or 75°C, and the products measured with qPCR. As depicted in FIG. 13G, there was no substantial increase in the amount of product where double-stranded splint ligation was performed with a pre-ligation incubation at either room temperature or 75°C.

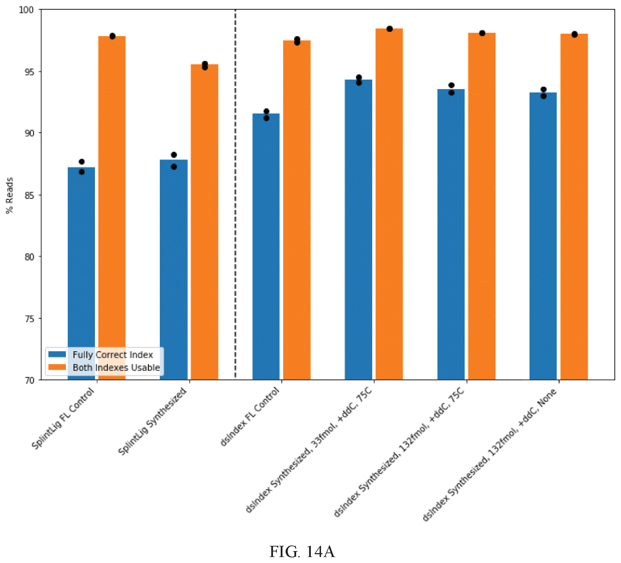

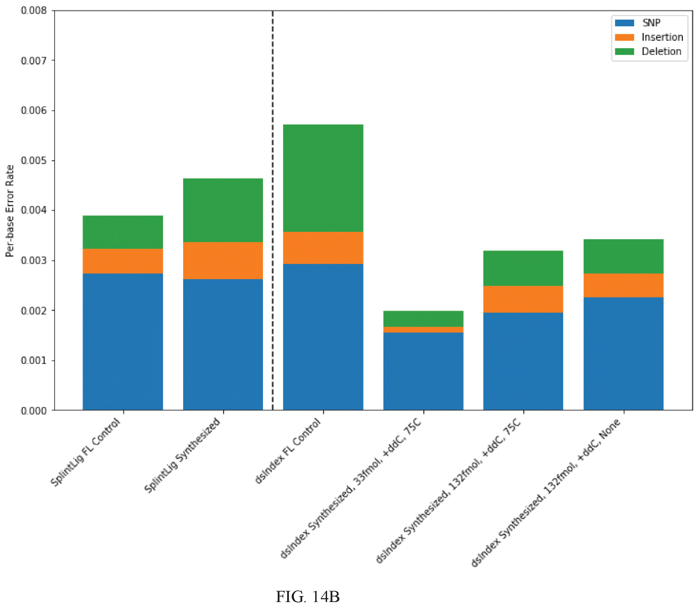

Direct beadcode sequencing for a 2-level double-stranded index ligation was performed. The results are summarized in FIG 14A and FIG. 14B.

From the foregoing 2-level experiments, double-stranded index ligation gave results similar to splint ligation as measured by qPCR. For double-stranded index amounts above 33 fmol/μg beads there was no substantial increase in full-length product with a single ligation or increase in errors within the beadcode. The following manipulations had little effect: (1) addition of an oligo complementary to index1; (2) removal of the 3' ddC modification; or (3) replacement of pre-ligation incubation at 75°C with room temperature incubation. In addition, beadcode sequencing showed fewer errors with double-stranded ligation compared to splint ligation.

Example 7-3-level indexing with double-stranded splint ligation

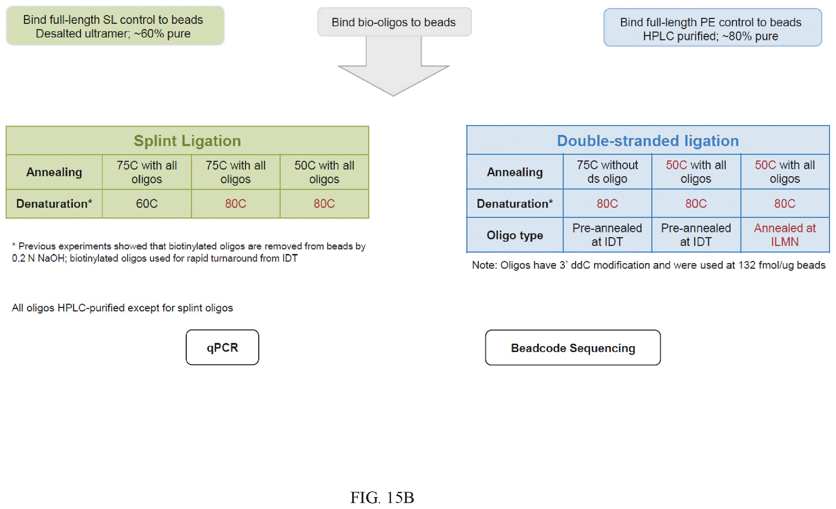

3-level indexing comprising double-stranded splint ligation was compared to 3-level indexing comprising splint ligation (standard). An overview of an experimental design is shown in FIG. 15A and FIG. 15B.

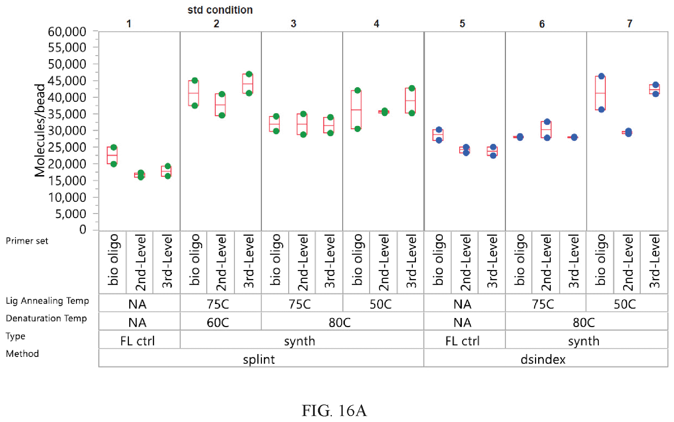

qPCR was performed on 3-level indexed products using either double-stranded splint ligation or splint ligation. Full-length double-stranded ligation index control was used as standard curve (higher purity), and concentration of splint ligation amplicons were size-adjusted. Results are summarized in FIG. 16A. For splint ligation, denaturation at 80°C reduced total number of oligos (2 vs 3), and annealing at 50°C slightly increased the total number of oligos (3 vs 4). For

splint ligation vs ds ligation, annealing at 50°C and denaturation at 80C gave comparable numbers of full-length oligos with splint and ds ligation methods (4 vs 7), oligos were full-length (3rd-level vs bio-oligo), and ds ligation showed a lower number of 2nd level oligos. For full-length controls, there was a lower total number of molecules, perhaps due to purity issues.

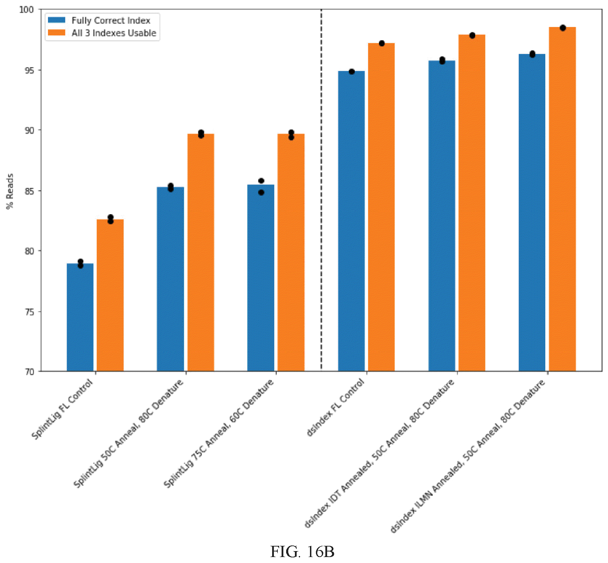

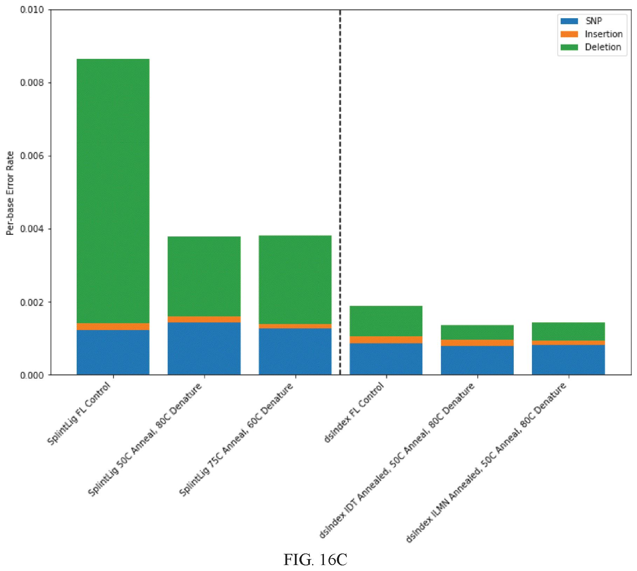

Direct beadcode sequencing for a 3-level double-stranded index ligation was performed. The results are summarized in FIG 16B and FIG. 16C.

From the foregoing double-stranded splint ligation studies, splint ligation and ds ligation gave comparable numbers of full-length oligos, and denaturation at 80°C led to loss of some capture oligonucleotides (COs), but annealing at 50°C slightly increased number of COs. COs synthesized by ds ligation had fewer errors than COs synthesized by splint ligation.

Example 8-LNA-containing splint oligos

Shortened first splints (kangaroo splints) are designed containing lock nucleic acid nucleotides (LNAs). The kangaroo splints are based on the sequence: (SEQ ID NO:03) ATGCTCTAGACAAGT/3ddC in which '3ddC' is 3' dideoxycytidine. Generated splints include those shortened from both the 5' end and the 3' end, all keeping the last base a dideoxy-cytidine (ddC), and those containing all possible LNA substitutions. Generated oligos are analyzed with IDT's OligoAnalyzer (Integrated DNA Technologies™, Coralville, Iowa). Generated oligos are selected that follow Qiagen's design guidelines for LNA oligos including: a GC content between 30-60%; <4 LNA bases in a row; <3 Gs or Cs in a row. Generated oligos are selected that have Tm's similar to original splint and do not have any LNAs at base positions complementary to ligation site. Generated oligos are selected that have no secondary structure or self-hybridization at room temperature, and are not complementary to P5 or the Hyb sequence. TABLE 2 lists three example oligos.

TABLE 2

| Name | Original kangaroo splint | k_lna_96 | k_lna_41 | k_lna_76 |
|---|---|---|---|---|
| Sequence (SEQ ID NO) | ATGCTCTAGACAAGT/3ddC/<br><br>ATGCTCTAGACAAGTC (SEQ ID NO:03) | TGC+T+CTA+C+AAG/3ddC/<br><br>TGCTCTACAAGC (SEQ ID NO:04) | +TG+CT+CTA+C+AAG/3ddC/<br><br>TGCTCTACAAGC (SEQ ID NO:05) | GCT+C+TAA+C+AAG/3ddC/<br><br>GCTCTAACAAGC (SEQ ID NO:06) |
| Length | 16 | 12 | 12 | 12 |
| GC content | 43.8 | 50 | 50 | 50 |
| Number of LNAs | 0 | 4 | 5 | 4 |
| 5'TM | 30.3 | 36.3 | 36.6 | 34.1 |
| 3'TM | 32.6 | 35.9 | 35.9 | 35.9 |
| Full length TM | 46.5 | 53.8 | 54 | 51.9 |
| Nucleotides to the left of "+" symbol are LNA nucleotides | | | | |

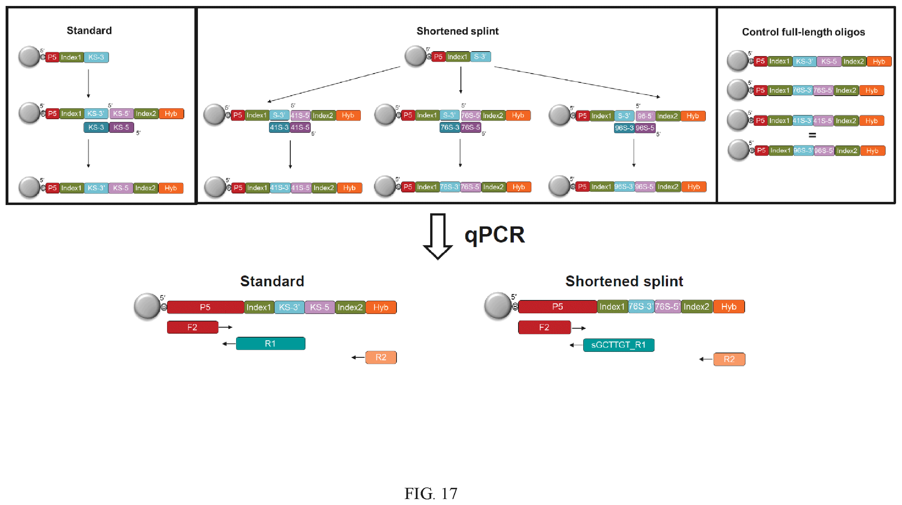

5' and 3' TMs are calculated using IDT's oligo analyzer with the following settings (based on ligation reaction): 1.98 μM oligo, 100 mM $Na^+$, 7.9 mM $Mg^{++}$. Full-length TMs are calculated using IDT's oligo analyzer with the following settings (based on splint denaturation): 0.05 μM oligo, 100 mM $Na^+$. An overview of the experiment is shown in FIG. 17.

Example 9-Modified splint oligonucleotides to reduce linker sequence lengths

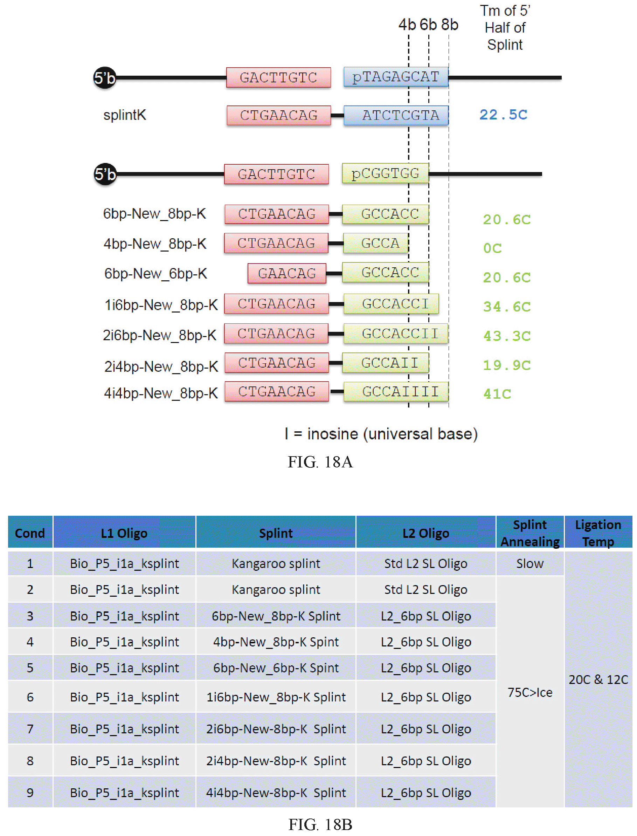

Further splints were designed in which a Phos-oligo splint hybridization sequence was decreased to 6 nucleotides and the G/C content was increased. Multiple splints were designed against the new phos-oligo. An overview of the designed splints is shown in FIG. 18A which shows the sequences listed in TABLE 3, where X is inosine.

TABLE 3

| SEQ ID NO: | SEQUENCE |
|---|---|
| SEQ ID NO:07 | GACTTGTCCGGTGG |
| SEQ ID NO:08 | CTGAACAGGCCACC |
| SEQ ID NO:09 | CTGAACAGGCCA |
| SEQ ID NO:10 | GAACAGGCCACC |
| SEQ ID NO:11 | CTGAACAGGCCACCX |
| SEQ ID NO:12 | CTGAACAGGCCACCXX |
| SEQ ID NO:13 | CTGAACAGGCCAXX |
| SEQ ID NO:14 | CTGAACAGGCCAXXXX |

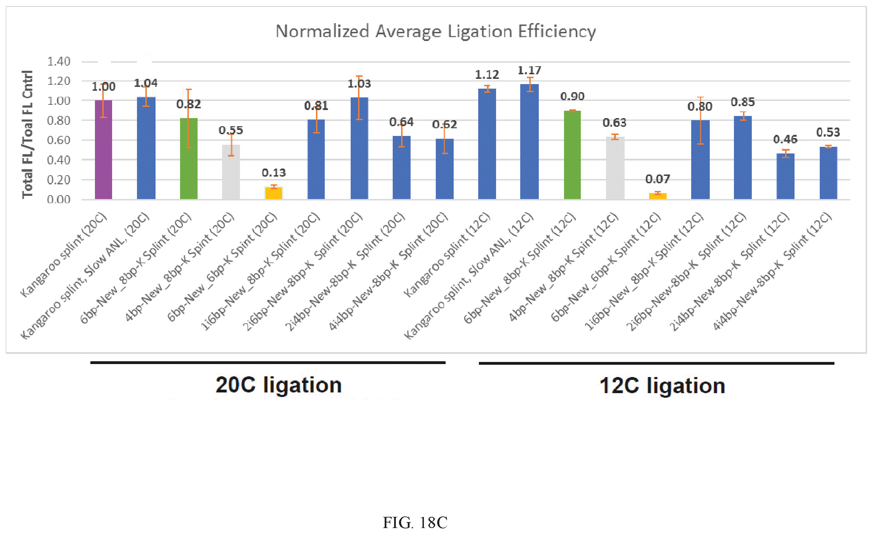

A summary of the experimental conditions is shown in FIG. 18B. Tested conditions included 20°C vs 12°C splint ligation which were carried out overnight in a thermocycler (no rotation). DS oligo annealing was performed using either a snap-cooling protocol (incubation at 75°C, immediately transferred to ice) or slow annealing (samples incubated at 75°C in a thermocycler, then temperature was ramped down with a decrease rate of 1°C/30s). qPCR measured capture and full-length oligo. Results are summarized in FIG. 18C which shows that there was about 10-20% less ligation with redesigned 6 nucleotide p-oligo/splint. There was about 40-50% less ligation with redesigned 4 nucleotide splint. From results with the ’6bp_New_6bp-K splint’ shown in FIG. 18A which had the shortest 3’ portion of the splint of the splints tested, it was inferred that reducing the 3’ half of splint to just 6 nucleotides reduced ligation efficiency ~90%. Slow annealing had little effect on number of full-length oligos. Adding inosine bases may not have improved ligation efficiency. Replacing bases with inosine may have reduced ligation efficiency. In summary, redesigning splint sequence and shortening the 5’ end of the splint to 6 nucleotides and 4 nucleotides reduced ligation efficiency, even when ligation was performed below the splint Tm. Adding inosines to the 5’ end of the splint did not improve ligation efficiency. Replacing bases at the 5’ end of the splint with inosines reduced ligation efficiency. Slow annealing was found to have little effect on ligation efficiency. A reduction in ligation efficiencies in preparing beads-linked indexed oligonucleotides with shorted linker sequences should likely be outweighed by increased efficiencies of sequencing workflows which include the use of linker sequences with reduced lengths.

Example 10-Workflow for indexing with double-stranded splint ligation

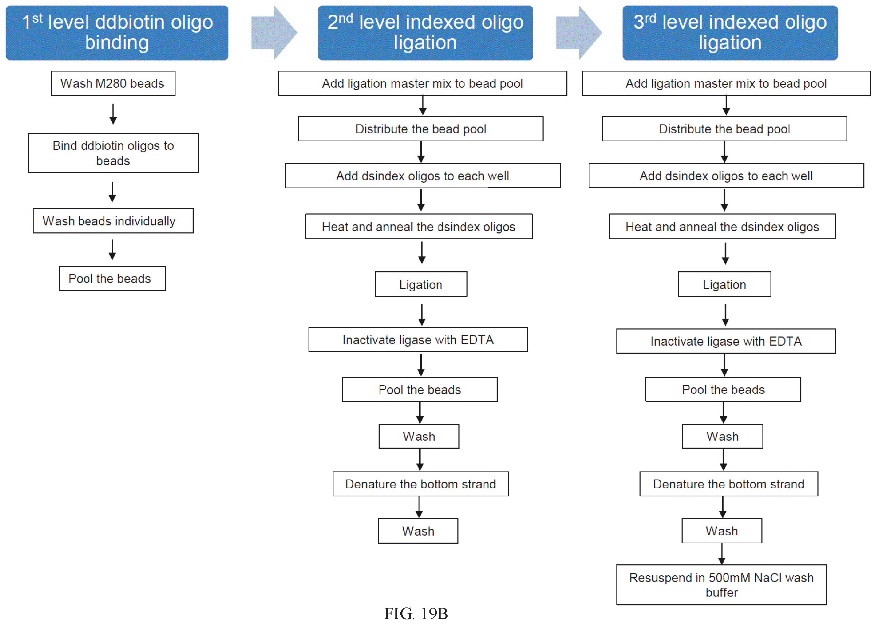

An overview of indexing with double-stranded splint ligation is shown in FIG. 19A and an example workflow is shown in FIG. 19B. In the scheme depicted in FIG. 19A, annealing includes: 60°C for 3min, transfer to ice; the ligating includes: 1st ligation: O/N, and 2nd ligation: 5hrs; and the denaturing include 80°C for 2 min, remove buffer immediately. The following summarizes steps for preparing a beadpool indexed by double-stranded splint ligation.

Oligo preparation

| Lyophilized oligos |
|---|
| If not in solution, suspend in RS1 (1x TE) to 100uM concentration. |
| For Index A oligos, prepare a 1uM working stock in a 96-well plate. |
| Recommended oligo storage @ 4C. |
| Dilute ds-SL oligos |
| Make fresh working dilutions day of bead pool prep. |

| Index A: 96 DDTB Oligos | Index B: 24 Indices | Index C: 24 Indices |
|---|---|---|
| For 250nM dilution: | For 5uM dilution: | For 5uM dilution: |
| 7.5ul 1x TE | 28.5ul 1x TE | 28.5ul 1x TE |

(continued)

| Index A: 96 DDTB Oligos | Index B: 24 Indices | Index C: 24 Indices |
|---|---|---|
| 2.5ul 1uM Working stock | 1.5ul 100uM stock | 1.5ul 100uM stock |

Starting M280 bead quantification

| Thoroughly mix stock of M280 beads before pipetting. |
|---|
| Make a 1000x dilution of M280 beads w/ TWB |
| - 25x: 5uL M280 in 120uL TWB |
| - 1000x: 4uL of 25x dilution in 156uL TWB |
| Measure beads/uL on Countess |

| Beads/uL | 752,500 |
|---|---|
| ug beads/uL (advertised) | 10 |
| Beads/ug (calc) | 75,250 |
| fmoles/mole | 1.00E+15 |
| molecules/mole | 6.02E+23 |
| Target molecules/bead (calc) | 48,016 |

DDTB-oligo binding (96-plex)

| **Day 1** |
|---|
| Take 1225uL of 10mg/mL M280 streptavidin beads into a eppindorf tube. |
| Place on magnet and allow beads to pellet. |
| Remove and discard supernatant. |
| Wash bead pellet with 1000uL of TWB 1x: |
| *- Pipette 1000uL TWB onto beads.* |
| *- Move tube back and forth on magnet 8x so beads travel through the buffer* |
| *- Remove and discard supernatant* |
| Resuspend beads in 1225uL TWB. Transfer to a trough. |
| Dilute beads to 2.5mg/mL with 3675uL of TWB. Pippet up and down to fully suspend beads. |
| Distribute 50uL beads/well into a 96 well plate. |
| Add indicated volume of a unique Index A oligo/well using a multi-P10. After addition to each column, pipette 8x w/ multi-P200. |
| Amount 250nM Index A/Well |
| Seal plate. Incubate at RT for 30min on rotator |
| Place samples on a magnet and allow the beads to pellet. |
| Remove and discard supernatant. |
| Wash bead pellet with 100uL of TWB 3x |

(continued)

| Day 1 |
| --- |
| Resuspend beads in 30uL of TWB/well. |
| Pool all beads in trough. |
| Wash plate where binding took place with 100uL TWB |
| *- Add TWB to wells in a single column. Pipette mix. Transfer wash to each column in plate.* |
| Combine plate wash with pooled beads. |
| Transfer beads to 5mL tube. |

1st splint ligation (24-plex)

| Prepare the following ligation MM: |
|---|

| LIG MM | Per Index | 24 Indicies |
|---|---|---|
| Reagent | 1x MM | 24.5x MM |
| H2O | 85.76 | 2101.0 |
| 10x E.coli Ligase Buffer | 9.44 | 231.4 |
| 5M NaCl | 2.40 | 58.8 |
| Total volume | 97.6 | 2391 |

| Remove TWB from beads, add 2391uL of LIG MM. |
|---|
| Pipet to mix, transfer to trough. |
| Distribute 97.6uL beads/well into a new 96 well plate. |
| - Roughly split excess beads between wells. |
| Add the following amount of 5uM Index B oligo/well: |
| Amount 5uM Index B/Well |
| Mix wells by pippette. Ensure beads are fully suspended. |
| Seal plate. Heat for 3m @ 60C then transfer to ice (~5 min). |
| To each well, add 10.4uL E.coli ligase (10U/uL). |
| Mix by pippette. Ensure beads are fully suspended. |
| Seal plate. Incubate at RT on rotator ~15hrs |
| **Day 2** |
| Inactivate ligase by adding 12uL of 500mM EDTA/well (MgCl2 conc. ~8mM, final EDTA conc. ~50mM). |
| Mix by pipetting. |
| Incubate at RT 5min to ensure all Mg is captured by EDTA. |
| Wash 2x with 100uL TWB + 10mM EDTA. |
| Wash 1x with 100uL TWB buffer. |
| Add 100uL TWB. Mix by pipette to fully suspend beads. |
| Seal plate. In thermocycler, heat at 80C for 2 min. |

| Remove TWB as quickly as possible. |
|---|
| Wash 1x with 100ul TWB. |
| Resuspend beads in 100uL of TWB/well. |
| Pool all beads in trough. |
| Wash plate where binding took place with 100uL TWB |
| - *Add TWB to wells in a single column. Pipette mix. Transfer wash to each column in plate.* |
| Combine wash with rest of pooled beads. |
| Transfer pooled beads to 5mL tube. |

2nd splint ligation (24-plex)

| Prepare the following ligation MM: | | |
|---|---|---|
| LIG MM | Per Index | Per Plate |
| Reagent | 1x MM | 24.5x MM |
| H2O | 85.76 | 2101.0 |
| 10x E.coli Ligase Buffer | 9.44 | 231.4 |
| 5M NaCl | 2.40 | 58.8 |
| Total volume | 97.6 | 2391 |
| Remove TWB from beads, add 2391uL of LIG MM. | | |
| Pipet to mix, transfer to trough. | | |
| Distribute 97.6uL beads/well into a new 96 well plate. | | |
| - Roughly split excess beads between wells. | | |
| Add following amount of 5uM Index B oligo/well: | | |
| Amount 5uM Index C/Well | | |
| Mix wells by pippette. Ensure beads are fully suspended. | | |
| Seal plate. Heat for 3m @ 60C then transfer to ice (~5 min). | | |
| To each well, add 10.4uL E.coli ligase (10U/uL). | | |
| Mix by pippette. Ensure beads are fully suspended. | | |
| Seal plate. Incubate at RT on rotator ~5hrs. | | |

| Inactivate ligase by adding 12uL of 500mM EDTA/well (MgCl2 conc. ~8mM, final EDTA conc. ~50mM). |
|---|
| Mix by pipetting. |
| Incubate at RT 5min to ensure all Mg is captured by EDTA. |
| Wash 2x with 100uL TWB + 10mM EDTA. |
| Wash 1x with 100uL TWB buffer. |
| Add 100uL TWB. Mix by pipette to fully suspend beads. |
| Seal plate. In thermocycler, heat at 80C for 2 min. |
| Remove TWB as quickly as possible. |
| Wash 1x with 100ul TWB. |
| Resuspend beads in 100uL of TWB/well. |
| Pool all beads in trough. |
| Wash plate where binding took place with 100uL TWB |
| - *Add TWB to wells in a single column. Pipette mix. Transfer wash to each column in plate.* |
| Combine wash with rest of pooled beads. |
| Transfer pooled beads to 1.5mL tube. |
| Resuspend beadpool in 1.2mL of 500mM NaCl Wash Buffer (Final conc ~10mg/mL) |

Final M280 bead quantification

| Make a 1000x dilution of M280 w/ TWB |
|---|
| - 25x: 5uL M280 in 120uL TWB |
| - 1000x: 4uL of 25x dilution in 156uL TWB |
| Measure beads/uL on Countess |

The following summarizes steps for a quantitative PCR (qPCR).

| **Prepare Sample Dilutions:** |
|---|
| Prepare 1000x dilution: |
| - Add 3uL sample in 147uL RSBT (50x). |
| - Add 5uL of 50x dilution in 95uL RSBT (1000x) |
| - Use countess to measure bead count of 1000x qPCR dilution (during/after qPCR) |

| **Preparing qPCR Standard Curve:** |
|---|
| Dilute 100uM std curve oligo to 1uM in RSBT (Add 2uL in 198uL RSBT). |
| - Store 1uM stock @ -20C for future use. |

(continued)

| **Preparing qPCR Standard Curve:** |
|---|
| Dilute 1uM std curve oligo to 20nM (Add 2uL in 98uL RSBT) |
| Prepare std curve by performing six 10x serial dilutions of the 20nM oligo (Std 1 = 2nM, Std 2 = 0.2nM, etc). |
| - 10ul previous dilution + 90ul RBST |
| Include a NTC in standard curve (Std 7) |

| **Prepare qPCR plate -** |
|---|
| Prepare qPCR MM (*see below).* |
| Pippete MM up and down to prime tips, before adding 16uL to wells of 384-well qPCR plate. |
| Add 4uL sample to 16uL of MM in qPCR plate. Pipette up and down 5x to mix. |
| Seal, vortex, and spin down qPCR plate at ~500rpm for ~5 sec (visually check there is no liquid stuck to top/clear seal) |
| Run qPCR program below (1.5 hrs total) |
| - Denature: 95C for 3m |
| - 30 Cycles: 95C for 5s, 55C for 30s, plate read |
| - Melt Curve: 55C > 95C |

The term "comprising" as used herein is synonymous with "including," "containing," or "characterized by," and is inclusive or open-ended and does not exclude additional, unrecited elements or method steps.

The above description discloses several methods and materials of the present invention. This invention is susceptible to modifications in the methods and materials, as well as alterations in the fabrication methods and equipment. Such modifications will become apparent to those skilled in the art from a consideration of this disclosure or practice of the invention disclosed herein. Consequently, it is not intended that this invention be limited to the specific embodiments disclosed herein, but that it cover all modifications and alternatives coming within the true scope and spirit of the invention.

All references cited herein, including but not limited to published and unpublished applications, patents, and literature references, are incorporated herein by reference in their entirety and are hereby made a part of this specification. To the extent publications and patents or patent applications incorporated by reference contradict the disclosure contained in the specification, the specification is intended to supersede and/or take precedence over any such contradictory material.

The invention is also described in the following clauses:

1. A method of preparing a plurality of combinatorially indexed beads, comprising:

(a) obtaining a population of primary indexed beads comprising a first polynucleotide comprising a first index, wherein the population of primary indexed beads comprises a plurality of first subpopulations of indexed beads, wherein the first subpopulations of indexed beads comprise a different first index from one another;
(b) splitting the population of primary indexed beads into a plurality of second subpopulations of beads;
(c) obtaining a population of secondary indexed beads, comprising:

(i) extending the first polynucleotide of the plurality of second subpopulations of beads with a second polynucleotide comprising a second index to obtain second subpopulations of indexed beads, wherein the second subpopulations of indexed beads comprise a different second index from one another, and
(ii) combining the second subpopulations of indexed beads to obtain the population of secondary indexed beads; and optionally, further comprising:

(d) splitting the population of secondary indexed beads into a plurality of third subpopulations of beads; and
(e) obtaining a population of tertiary indexed beads, comprising:

(i) extending the second polynucleotide of the plurality of third subpopulations of beads with a third polynucleotide comprising a third index to obtain third subpopulations of indexed beads, wherein the third

subpopulations of indexed beads comprise a different third index from one another, and
(ii) combining the third subpopulations of indexed beads to obtain the population of tertiary indexed beads.

2. The method of clause 1, wherein (c) comprises extending the first polynucleotide with the second polynucleotide by chemical ligation.
3. The method of clause 2, wherein:

the first polynucleotide comprises a terminal 3' modified deoxynucleotide (dNTP) comprising a 3' functional moiety capable of participating in a click chemistry reaction;
the second polynucleotide comprises a terminal 5' modified dNTP comprising a compatible 5' functional moiety capable of participating in a click chemistry reaction with the 3'-functional moiety; and
the 3' functional moiety and the 5' functional moiety are capable of reacting with one another to form a modified backbone linkage.

4. The method of clause 3, wherein the extending the first polynucleotide with the second polynucleotide obtains a secondary indexed polynucleotide, and the method further comprises modifying the secondary indexed polynucleotide to obtain a modified polynucleotide comprising a terminal 3' modified deoxynucleotide (dNTP) comprising a 3' functional moiety capable of participating in a click chemistry reaction.
5. The method of clause 4, wherein the modifying comprises contacting the secondary indexed polynucleotide with a template independent polymerase.
6. The method of clause 5, wherein the template independent polymerase is selected from a terminal deoxynucleotidyl transferase (TdT), PolyA polymerase, or CCA-adding RNA polymerase.
7. The method of clause 6, wherein the template independent polymerase is TdT.
8. The method of any one of clauses 3-7, further comprising extending the modified polynucleotide with the third polynucleotide by a chemical ligation reaction, wherein the third polynucleotide comprises a terminal 5' modified dNTP comprising a compatible 5' functional moiety capable of participating in a click chemistry reaction with the 3' functional moiety.
9. The method of any one of clauses 3-8, wherein the 3' functional moiety is selected from the group consisting of an azide, an alkynyl, an alkenyl, a thiol, and a nitrone.
10. The method of any one of clauses 3-9, wherein the 5' functional moiety is different from and compatible with the 3' functional moiety and is selected from the group consisting of an azide, an alkynyl, an alkenyl, a thiol, and a nitrone
11. The method of any one of clauses 3-10, wherein the 3' functional moiety and the 5' functional moiety are selected from the following pairs: (i) 3'-azido/5'-alkynyl; (ii) 3'-alkynyl/5' azido; (iii) 3'-thiol/5'-alkynyl; (iv) 3'-thiol/5'-alkenyl; (v) 3'-alkynyl/5'-thiol; (vi) 3'-alkenyl/5'-thiol; (vii) 3'-azido/5'-cyclooctynyl; (viii) 3'-cyclooctyne/5'-azido; (ix) 3'-nitrone/5'-cyclooctynyl; and (x) 3'-cyclooctynyl/5'-nitrone.
12. The method of any one of clauses 3-11, wherein the 3' functional moiety is a 3'-azido and the 5' functional moiety is a 5'-alkynyl.
13. The method of any one of clauses 3-12, wherein the click chemistry reaction comprises copper catalyzed azide-alkyne cycloaddition (CuAAC) to form a modified backbone linkage comprising a triazolyl.
14. The method of clause 1, wherein (c) comprises extending the first polynucleotide by polymerase extension, wherein the first polynucleotide comprises a first linker.
15. The method of clause 14, further comprising:

(i) obtaining a first adaptor comprising a region capable of hybridizing to the first linker and a region comprising the second index or complement of the second index;
(ii) hybridizing the first adaptor to the first linker; and
(iii) extending the first polynucleotide to obtain a secondary indexed polynucleotide.

16. The method of clause 14 or 15, wherein the first adaptor comprises a non-extendable 3' end.
17. The method of clause 16, wherein the non-extendable 3' end comprises a 3'2' dideoxy nucleotide, or a C3 linker.
18. The method of any one of clauses 14-17, further comprising removing the first adaptor from the secondary indexed polynucleotide.
19. The method of clause 18, wherein the removing comprises denaturing the first adaptor by heat or base, or degrading the first adaptor by enzymic degradation.
20. The method of any one of clauses 14-19, wherein the first linker has a length less than 10 consecutive nucleotides.
21. The method of any one of clauses 14-20, wherein the first linker has a length less than 5 consecutive nucleotides.
22. The method of any one of clauses 14-21, wherein (e) comprises extending the second polynucleotide by polymerase extension.

23. The method of any one of clauses 15-22, wherein the first adaptor comprises a complement of a second linker, such that the secondary indexed polynucleotide comprises the second linker.

24. The method of clause 23, further comprising:

(i) obtaining a second adaptor comprising a region capable of hybridizing to the second linker and a region comprising the third index or complement of the third index;
(ii) hybridizing the second adaptor to the second linker; and
(iii) extending the secondary indexed polynucleotide to obtain a tertiary indexed polynucleotide.

25. The method of clause 24, wherein the second adaptor comprises a non-extendable 3' end.

26. The method of clause 25, wherein the non-extendable 3' end comprises a 3'2' dideoxy nucleotide, or a C3 linker.

27. The method of any one of clauses 24-26, further comprising removing the second adaptor from the tertiary indexed polynucleotide.

28. The method of clause 27, wherein the removing comprises denaturing the first adaptor by heat or base, or degrading the first adaptor by enzymic degradation.

29. The method of any one of clauses 23-28, wherein the second linker has a length less than 10 consecutive nucleotides.

30. The method of any one of clauses 23-29, wherein the second linker has a length less than 5 consecutive nucleotides.

31. The method of clause 1, wherein (c) comprises extending the first polynucleotide with the second polynucleotide by ligation.

32. The method of clause 31, further comprising:

(i) obtaining a double stranded first adaptor comprising the second polynucleotide and a 3' single stranded overhang capable of hybridizing to a first linker of the first polynucleotide;
(ii) hybridizing the first adaptor to the first linker, and optionally, hybridizing an additional oligonucleotide to the first index; and
(iii) ligating the first polynucleotide to the second polynucleotide to obtain a secondary indexed polynucleotide.

33. The method of clause 32, further comprising extending the second polynucleotide with a third polynucleotide by ligation.

34. The method of clause 33, further comprising:

(i) obtaining a double stranded second adaptor comprising the third polynucleotide and a 3' single stranded overhang capable of hybridizing to a second linker of the second polynucleotide;
(ii) hybridizing the second adaptor to the second linker; and
(iii) ligating the second polynucleotide to the third polynucleotide to obtain a tertiary indexed polynucleotide.

35. The method of any one of clauses 31-34, wherein the ligation comprises use of a ligase.

36. The method of any one of clauses 31-34, wherein the ligation comprises a chemical ligation reaction.

37. The method of clause 1, wherein (c) comprises extending the first polynucleotide by ligation, wherein the first polynucleotide comprises a first linker, and the second polynucleotide comprises a second linker.

38. The method of clause 37, further comprising:

(i) obtaining a first adaptor comprising a region capable of hybridizing to the first linker and a region capable of hybridizing to the second linker;
(ii) hybridizing the first adaptor to the first linker;
(iii) hybridizing the second oligonucleotide to the region capable of hybridizing to the second linker; and
(iv) ligating the first polynucleotide to the second polynucleotide to obtain a secondary indexed polynucleotide.

39. The method of clause 38, wherein the first linker and/or the second linker has a length less than 10 consecutive nucleotides.

40. The method of clause 39, wherein the first linker and/or the second linker has a length less than 5 consecutive nucleotides.

41. The method of any one of clauses 37-40, wherein the first linker and/or the second linker is modified to have an increased $T_m$ compared to an oligonucleotide having the same length as the first adaptor.

42. The method of clause 41, wherein the first linker and/or the second linker comprises an increased G/C content compared to the oligonucleotide having the same length, or comprises modified nucleotides.

43. The method of any one of clauses 37-42, further comprising removing the first adaptor from the secondary indexed polynucleotide.
44. The method of clause 43, wherein the removing comprises denaturing the first adaptor by heat or base, or degrading the first adaptor by enzymic degradation.
45. The method of any one of clauses 37-44, wherein (e) comprises extending the second polynucleotide by ligation, wherein the second oligonucleotide comprises a third linker, such that the secondary indexed polynucleotide comprises the third linker, and wherein the third polynucleotide comprises a fourth linker.
46. The method of clause 45, further comprising:

(i) obtaining a second adaptor comprising a region capable of hybridizing to the third linker and a region capable of hybridizing to the fourth linker;
(ii) hybridizing the second adaptor to the third linker;
(iii) hybridizing the third polynucleotide to the second adaptor via the region capable of hybridizing to the fourth index; and
(iv) ligating the secondary indexed polynucleotide to the third polynucleotide to obtain a tertiary indexed polynucleotide.

47. The method of clause 46, wherein the third linker and/or the fourth linker has a length less than 9 consecutive nucleotides.
48. The method of clause 47, wherein the third linker and/or the fourth linker has a length less than 5 consecutive nucleotides.
49. The method of any one of clauses 45-48, wherein the third linker and/or the fourth linker is modified to have an increased $T_m$ compared to an oligonucleotide having the same length as the second adaptor.
50. The method of clause 49, wherein the third linker and/or the fourth linker comprises an increased G/C content compared to the oligonucleotide having the same length, or comprises modified nucleotides.
51. The method of any one of clauses 46-50, further comprising removing the second adaptor from the tertiary indexed polynucleotide.
52. The method of clause 51, wherein the removing comprises denaturing the first adaptor by heat or base, or degrading the first adaptor by enzymic degradation.
53. The method of any one of clauses 1-52, wherein (a) comprises:

(i) attaching the first polynucleotide to a plurality of first subpopulations of beads to obtain the first subpopulations of indexed beads, wherein the first polynucleotide comprises a different first index for each first subpopulation of beads; and
(ii) combining the first subpopulations of indexed beads to obtain the population of primary indexed beads.

54. The method of clause 53, wherein the first polynucleotide is attached to a bead via a first binding partner and a second binding partner.
55. The method of clause 54, wherein the first binding partner or the second binding partner is selected from the group consisting of biotin, streptavidin, a biotin derivative, a streptavidin derivative, an antibody, and an antigen binding fragment of an antibody.
56. The method of any one of clauses 1-55, further comprising repeating (d) and (e) and adding additional indexes to indexed subpopulations of beads.
57. The method of any one of clauses 1-56, wherein the first polynucleotide comprises a primer binding site selected from the group consisting of a P5 sequence, a P5' sequence, P7 sequence, and P7' sequence.
58. The method of any one of clauses 1-57, wherein the first index, the second index, and/or the third index has a length less than 20 consecutive nucleotides.
59. The method of any one of clauses 1-58, wherein the first index, the second index, and/or the third index has a length less than 10 consecutive nucleotides.
60. The method of any one of clauses 1-59, wherein (b) comprises randomly distributing the population of primary indexed beads into a plurality of compartments.
61. The method of any one of clauses 1-60, wherein (d) comprises randomly distributing the population of secondary indexed beads into a plurality of compartments.
62. The method of clause 60 or 61, wherein the plurality of compartments comprise a compartment selected from a well, a channel, or a droplet.
63. The method of any one of clauses 1-62, wherein the plurality of combinatorially indexed beads comprise magnetic beads.
64. The method of any one of clauses 1-63, further comprising distributing the plurality of combinatorially indexed

beads on an array.

65. The method of any one of clauses 1-64, further comprising sequencing the combinatorially indexed beads on an array.

66. The method of clause 65, further comprising decoding the location of a bead of the plurality of combinatorially indexed beads on an array based on the combinatorial indexes.

67. The method of any one of clauses 1-66, wherein each bead of the plurality of combinatorially indexed beads comprises a capture probe.

68. The method of clause 67, wherein the first polynucleotide, the second polynucleotide or the third polynucleotide comprises the capture probe.

69. The method of any one of clauses 1-68, further comprising hybridizing a plurality of target nucleic acid to the capture probes.

70. The method of clause 69, further comprising extending the capture probes.

## Claims

**1.** A method of preparing a plurality of combinatorially indexed beads, comprising:

(a) obtaining a population of primary indexed beads comprising a first polynucleotide comprising a first index and a first linker, wherein the population of primary indexed beads comprises a plurality of first subpopulations of indexed beads, wherein the first subpopulations of indexed beads comprise a different first index from one another;
(b) splitting the population of primary indexed beads into a plurality of second subpopulations of beads;
(c) obtaining a population of secondary indexed beads, comprising:

(i) obtaining a first adaptor comprising a region capable of hybridizing to the first linker and a region comprising a second index or complement of the second index;

hybridizing the first adaptor to the first linker; and
extending the first polynucleotide by polymerase extension to obtain a secondary indexed polynucleotide,
wherein the extending obtains a secondary indexed polynucleotide and second subpopulations of indexed beads,
wherein the second subpopulations of indexed beads comprise a different second index from one another, and

(ii) combining the second subpopulations of indexed beads to obtain the population of secondary indexed beads, thereby obtaining a plurality of combinatorially indexed beads.

**2.** The method of claim 1, wherein the first adaptor comprises a non-extendable 3' end.

**3.** The method of claim 2, wherein the non-extendable 3' end comprises a 3 '2' dideoxy nucleotide or a C3 linker.

**4.** The method of any one of claims 1-3, wherein the first linker has a length less than 10 consecutive nucleotides.

**5.** The method of any one of claims 1-4, wherein step (ii) further comprises removing the first adaptor from the secondary indexed polynucleotide.

**6.** The method of claim 5, wherein the removing comprises denaturing the first adaptor by heat or base, or degrading the first adaptor by enzymatic degradation.

**7.** The method of any one of claims 1-6, wherein the first polynucleotide comprises a 5' end linked to a biotin or a 5' dual desthiobiotin.

**8.** The method of any one of claims 1-7, wherein the first adaptor comprises a complement of a second linker, such that the secondary indexed polynucleotide comprises the second linker; optionally, wherein the method further comprises:

(i) obtaining a second adaptor comprising a region capable of hybridizing to the second linker and a region

comprising the third index or complement of the third index, optionally, wherein the second adaptor comprises a non-extendable 3' end;
(ii) hybridizing the second adaptor to the second linker; and
(iii) extending the secondary indexed polynucleotide to obtain a tertiary indexed polynucleotide.

**9.** The method of claim 8, wherein step (iii) comprises extending the second polynucleotide by polymerase extension.

**10.** The method of any one of claims 1-9, wherein step (a) comprises:

(i) attaching the first polynucleotide to a plurality of first subpopulations of beads to obtain the first subpopulations of indexed beads, wherein the first polynucleotide comprises a different first index for each first subpopulation of beads; and
(ii) combining the first subpopulations of indexed beads to obtain the population of primary indexed beads.

**11.** The method of any one of claim 1-10, wherein polymerase extension is performed with a T4 polymerase.

**12.** The method of any one of claims 1-11, wherein step (b) comprises randomly distributing the population of primary indexed beads into a plurality of compartments.

**13.** The method of any one of claims 1-12, further comprising distributing the plurality of combinatorially indexed beads on an array.

**14.** The method of any one of claims 1-13, further comprising sequencing the combinatorially indexed beads on an array.

**15.** The method of any one of claims 1-14, wherein each bead of the plurality of combinatorially indexed beads comprises a capture probe, and the method further comprises hybridizing a plurality of target nucleic acids to the capture probes.

A
B
C
D
E
F
1
2
3
4
5
6
7
8
Step 1:
Link oligo to bead through Bio-SA
Each well receives SA-beads and a unique oligo sequence (Index 1)
Step 2:
Pool all beads
Distribute bead pool across all wells
Step 3:
Attach oligo to bead-linked oligo
Each well receives a unique oligo sequence (Index B)
Step 4:
Pool all beads
Distribute pool across all wells
Step 5:
Ligate oligo to bead-linked oligo
Each well receives a unique oligo sequence (Index C)
Step 6:
Pool all beads
5'
B
P5
Index 1
Link 1
Index 2
Link 2
Index 3
Hyb


FIG. 1

5'
Index A
Link 1a
5'PO4
Link 1b
Index B
Link 2a
Link 1'
5'
Link 1'
5'
Index A
Link 1
Index B
Link 2a
5'PO4
Link 2b
Index C
Link 2
5'
Link 2'
5'
Index A
Link 1
Index B
Link 2
Index C

FIG. 2

P5
Index A
3
N3
5
Index B
3' Propargyl on capture oligo
5' Azide on index
Cu(I)-catalyzed
Click reaction
(CuAAC)
N
N=N
Adding additional index levels:
TdT
Don't want index oligo to have 3' alkyne initially to prevent formation of concatamers
A-3
For > 2 levels, indexes separated by a single base

| | 2-Level Dark Cycles | 3-Level Dark Cycles |
|---|---|---|
| Current | 16 | 32 |
| Chem Lig. | 0 | 0-1 |

FIG. 3

B
P5
Index A
Link 1a
Link 1a'
Index B'
Link 2a'
5'
B
P5
Index A
Link 1a
Index B
Link 2a
Link 2a'
Index C'
Hyb'
5'
B
P5
Index A
Link 1a
Index B
Link 2a
Index C
Hyb

FIG. 4

B
P5
Index A
Link 1a
Index B
Link 2a
Link 1a'
Index B'
5'
B
P5
Index A
Link 1a
Index B
Link 2a
Index C'
Hyb
Link 2a'
Index C'
5'
B
P5
Index A
Link 1a
Index B
Link 2a
Index C
Hyb

FIG. 5

5'PO4
Link 1b
Index B
Link 2a
B
P5
Index A
Link 1a
B
P5
Index A
Link 1
Index B
Link 2a
5'PO4
Link 2b
Index C
Hyb
B
P5
Index A
Link 1
Index B
Link 2
Index C
Hyb

FIG. 6

Capture Oligo
B
P5
Index A
Link 1a
Link 1a'
Index B'
Hyb'
5'
B
P5
Index A
Link 1a
Index B
Hyb
Full Length Product

FIG. 7A

Number of Molecules/Bead
Capture Oligos
Full Length Product
50000
40000
30000
20000
10000
0
1
2
3
Full length CTRL
POS CTRL
Full length CTRL
NEG CTRL
0 Exo-
330 Exo-
660 Exo-
0 Exo+
330 Exo+
Template (fmol/ug)
T4 Pol Enzyme
Ligation
Polymerase Extension

FIG. 7B

9
Bind full-length SL control to beads
Desalted ultramer; ~60% pure
2 & 10
Bind bio-oligos to beads
1
Bind full-length PE control to beads
HPLC purified; ~80% pure
Splint ligation
Polymerase extension
11
First ligation
• o/n 20C, no rotation
• Splint denatured at 60C for 2 min.
12
Second ligation
• 4h 20C, no rotation
• Splint denatured at 60C for 2 min.
5
First extension
• 15 min. 20C, no rotation
• Splint denatured with 0.2N NaOH
8
Second extension
• 15min. 20C, no rotation
• Splint denatured with 0.2N NaOH
First extension
6
Second extension
• No template
• No enzyme
First extension
7
Second extension
• No template
3
First extension
• No template
• No enzyme
4
First extension
• No template
qPCR
Beadcode Sequencing

FIG. 8A

Polymerase Extension | Splint Ligation

| Cond | 1 | 2 | 3 | 4 | 5 | 6 | 7 | 8 | 9 | 10 | 11 | 12 |
|---|---|---|---|---|---|---|---|---|---|---|---|---|
| 1st Rxn | No | No | Bfr | T4 | T4 | T4 | T4 | T4 | No | No | T7 | T7 |
| L1 | FL PE | CO | CO | CO | CO | CO | CO | CO | FL SL | CO | CO | CO |
| L2 | No | No | No | No | Yes | Yes | Yes | Yes | No | No | Yes | Yes |
| 2nd Rxn | No | No | No | No | No | Bfr | T4 | T4 | No | No | No | T7 |
| L3 | No | No | No | No | No | No | No | Yes | No | No | No | Yes |

FIG. 8B

Average Molecules/Bead
CO
2nd-Level
3rd-Level
Molecules/Bead
50,000
45,000
40,000
35,000
30,000
25,000
20,000
15,000
10,000
5,000
0
37,512
35,904
34,831
36,422
2
0
24,019
3
1
28,441
10
1
16,979
9,768
1
18,119
11,443
12
11,256
3,146
2
12,593
5,892
2,644
23,446
19,094
21,074
34,340
2
1
35,305
35,676
0
32,258
33,536
12,881
Cond 1 2 3 4 5 6 7 8 9 10 11 12
1st Rxn No No Bfr T4 T4 T4 T4 T4 No No T7 T7
L1 FL PE CO CO CO CO CO CO CO FL SL CO CO CO
L2 No No No No Yes Yes Yes Yes No No Yes Yes
2nd Rxn No No No No No Bfr T4 T4 No No No T7
L3 No No No No No No No Yes No No No Yes
No ext.
1 extension
2 extensions
Polymerase extension
No lig.
1 lig.
2 lig.
Splint ligation

FIG. 9

Concentration (nM) vs. Denaturation_Method & Bio_or_ddBio
Concentration (nM)
50
45
40
35
30
25
20
15
10
5
0
Bio
ddBio
None (- Ctl)
2xWashOnly
2x60C
2x80C
1x0.2N_NaOH
2x0.2N_NaOH

FIG. 10

Read Orientation:
Synthesized Product:
(on bead)
Synthesis
5
P5
Index1
S1
Index2
S2
Index3
Hyb
3
PCR Product:
5
P5
Index1
S1
Index2
S2
Index3
Hyb
SmpIdx'
ME_V2_B15'
P7'
3
ME_V2_B15
Sequencing
Sequencing Read:
5
SmpIdx'
Hyb
Index3
S2
Index2
S1
Index1
P5
3

FIG. 11A

100
95
90
85
80
75
70
% Reads
Fully Correct Index
Both Indexes Usable
PolExt FL Control
PolExt Synthesized
SplintLig FL Control
SplintLig Synthesized


FIG. 11B

SNP
Insertion
Deletion
Per-base Error Rate
0.014
0.012
0.010
0.008
0.006
0.004
0.002
0.000
PolExt FL Control
PolExt Synthesized
SplintLig FL Control
SplintLig Synthesized

FIG. 11C

Standard
ddBio_P5_i1a_ksplint
5'
B
P5
Index1
KS-3
L2 SL Oligo (CWB_o028)
KS-3'
KS-5'
Index2
Hyb
KS-3
KS-5
Kangaroo splint
ddBio ctrl full-length oligo (CWB_o029)
Double-stranded oligo
phx_030_index2_ddc
Tm during annealing: 45C
Tm during ligation: 41C
pre-annealed from IDT
Index2'
H'
phx_030_index1_bottom
(optional)
ddBio short full-length oligo (CWB_o027)

FIG. 12A

Bind full-length splint ligation control to beads
Splint ligation
Add master mix, splint oligo, and phos-oligo
Heat to 75C for 3min and cool on ice
Add T7 ligase
Incubate overnight at RT
qPCR
Bind ddbio-oligos to beads
ds oligo ligation
I.
Test 33, 66, 132, 264 fmol dsoligo/ ug beads
Test with or without 3' ddC
Add master mix
Heat to 75C for 3min and cool on ice
Add ds oligo and T7 ligase
Incubate overnight at RT
II.
132 fmol dsoligo/ ug beads
With 3' ddC
Add master mix
Add ds oligo and T7 ligase
Incubate overnight at RT
Sequencing
Bind full-length dsoligo control to beads
III.
132 fmol dsoligo/ ug beads
With 3' ddC
Add master mix and index1_bottom
Heat to 75C for 3min and cool on ice
Add ds oligo and T7 ligase
Incubate overnight at RT

FIG. 12B

standard

5'
B
P5
Index1
KS-3'
KS-5
Index2
Hyb
F2
R1
R2
F3

dsindex ligation

5'
B
P5
Index1
KS-3'
Index2
Hyb
F2
R1
R2
F3

FIG. 12C

Conc (nM)
100
90
80
70
60
50
40
30
20
10
0
NA
NA
splint
dsindex
splint
dsindex
splint
dsindex
splint
dsindex
splint
dsindex
splint
dsindex
Method
ddBio-PEstd
ddBio-SLstd
FL ctrl
synth
FL ctrl
synth
FL ctrl
synth
Type
ddbio oligo
FL-set1
FL-set2
Primer set

FIG. 13A

Normalized to FL controls
Conc relative to FL control
2
1.8
1.6
1.4
1.2
1
0.8
0.6
0.4
0.2
0
33fmol
33fmol
33fmol
33fmol
[Phos-Oligo]
FL-set1
FL-set2
FL-set1
FL-set2
Primer set
splint
dsindex
Method
synth
Type

FIG. 13B

Normalized to ddbio oligo concentration
Conc relative to ddbio oligo
1.2
1.1
1
0.9
0.8
0.7
0.6
0.5
0.4
0.3
0.2
0.1
0
ddbio oligo
FL-set1
FL-set2
ddbio oligo
FL-set1
FL-set2
ddbio oligo
FL-set1
FL-set2
ddbio oligo
FL-set1
FL-set2
Primer set
splint
dsindex
splint
dsindex
Method
FL ctrl
synth
Type

FIG. 13C

Conc (nM)
90
80
70
60
50
40
30
20
10
0
ddbio oligo
FL-set1
FL-set2
ddbio oligo
FL-set1
FL-set2
ddbio oligo
FL-set1
FL-set2
ddbio oligo
FL-set1
FL-set2
ddbio oligo
FL-set1
FL-set2
Primer set
33fmol
33fmol
66fmol
132fmol
264fmol
[Phos-Oligo]
splint
dsindex
Method
synth
Type


FIG. 13D

Conc (nM)
40
35
30
25
20
15
10
5
0
ddbio oligo
FL-set1
FL-set2
ddbio oligo
FL-set1
FL-set2
Primer set
dsindex
dsindex
Method
synth
synth
Type
-
index1
index1


FIG. 13E

Conc (nM)
50
45
40
35
30
25
20
15
10
5
0
ddbio oligo
FL-set1
FL-set2
ddbio oligo
FL-set1
FL-set2
Primer set
dsindex
dsindex
Method
synth
synth
Type
ddC
noddC
3ddC


FIG. 13F

Conc (nM)
50
45
40
35
30
25
20
15
10
5
0
ddbio oligo
FL-set1
FL-set2
ddbio oligo
FL-set1
FL-set2
Primer set
dsindex
dsindex
Method
synth
synth
Type
75C
RT
Pre-Lig Incubation


FIG. 13G

100
95
90
85
80
75
70
% Reads
Fully Correct Index
Both Indexes Usable
SplintLig FL Control
SplintLig Synthesized
dsIndex FL Control
dsIndex Synthesized, 33fmol, +ddC, 75C
dsIndex Synthesized, 132fmol, +ddC, 75C
dsIndex Synthesized, 132fmol, +ddC, None

FIG. 14A

SNP
Insertion
Deletion
Per-base Error Rate
0.008
0.007
0.006
0.005
0.004
0.003
0.002
0.001
0.000
SplintLig FL Control
SplintLig Synthesized
dsIndex FL Control
dsIndex Synthesized, 33fmol, +ddC, 75C
dsIndex Synthesized, 132fmol, +ddC, 75C
dsIndex Synthesized, 132fmol, +ddC, None

FIG. 14B

Standard
5'
P5
Index1
KS-3
KS-3'
KS-5'
Index2
MS-3'
KS-5
KS-5
MS-5
Index3
Hyb
Double-stranded oligo
Tm during annealing: 63C
Tm during ligation: 61C
Tm during denaturation: 61C
Index2'
MS-3
Tm during annealing: 70C
Tm during ligation: 70C
Tm during denaturation: 68C
Index3'
Hyb'

FIG. 15A

Bind full-length SL control to beads
Desalted ultramer; ~60% pure

Bind bio-oligos to beads

Bind full-length PE control to beads
HPLC purified; ~80% pure

| Splint Ligation | | | |
|---|---|---|---|
| **Annealing** | 75C with all oligos | 75C with all oligos | 50C with all oligos |
| **Denaturation*** | 60C | 80C | 80C |

* Previous experiments showed that biotinylated oligos are removed from beads by 0.2 N NaOH; biotinylated oligos used for rapid turnaround from IDT

All oligos HPLC-purified except for splint oligos

| Double-stranded ligation | | | |
|---|---|---|---|
| **Annealing** | 75C without ds oligo | 50C with all oligos | 50C with all oligos |
| **Denaturation*** | 80C | 80C | 80C |
| **Oligo type** | Pre-annealed at IDT | Pre-annealed at IDT | Annealed at ILMN |

Note: Oligos have 3' ddC modification and were used at 132 fmol/ug beads

**qPCR**

**Beadcode Sequencing**

FIG. 15B

std condition
1
2
3
4
5
6
7
Molecules/bead
60,000
55,000
50,000
45,000
40,000
35,000
30,000
25,000
20,000
15,000
10,000
5,000
0
Primer set
bio oligo
2nd-Level
3rd-Level
Lig Annealing Temp
NA
75C
75C
50C
NA
75C
50C
Denaturation Temp
NA
60C
80C
NA
80C
Type
FL ctrl
synth
FL ctrl
synth
Method
splint
dsindex

FIG. 16A

100
95
90
85
80
75
70
% Reads
Fully Correct Index
All 3 Indexes Usable
SplintLig FL Control
SplintLig 50C Anneal, 80C Denature
SplintLig 75C Anneal, 60C Denature
dsIndex FL Control
dsIndex IDT Annealed, 50C Anneal, 80C Denature
dsIndex ILMN Annealed, 50C Anneal, 80C Denature


FIG. 16B

0.010
0.008
0.006
0.004
0.002
0.000
Per-base Error Rate
SNP
Insertion
Deletion
SplintLig FL Control
SplintLig 50C Anneal, 80C Denature
SplintLig 75C Anneal, 60C Denature
dsIndex FL Control
dsIndex IDT Annealed, 50C Anneal, 80C Denature
dsIndex ILMN Annealed, 50C Anneal, 80C Denature


FIG. 16C

Standard
Shortened splint
Control full-length oligos
5'
P5
Index1
KS-3
KS-3'
KS-5'
KS-5
Index2
Hyb
S-3
S-3'
41S-3
41S-5
76S-3
76S-5
96S-3
96S-5
=
qPCR
F2
R1
R2
sGCTTGT_R1

FIG. 17

Tm of 5' Half of Splint
4b 6b 8b
5'b
GACTTGTC
pTAGAGCAT
splintK
CTGAACAG
ATCTCGTA
22.5C
5'b
GACTTGTC
pCGGTGG
6bp-New_8bp-K
CTGAACAG
GCCACC
20.6C
4bp-New_8bp-K
CTGAACAG
GCCA
0C
6bp-New_6bp-K
GAACAG
GCCACC
20.6C
1i6bp-New_8bp-K
CTGAACAG
GCCACCI
34.6C
2i6bp-New_8bp-K
CTGAACAG
GCCACCII
43.3C
2i4bp-New_8bp-K
CTGAACAG
GCCAII
19.9C
4i4bp-New_8bp-K
CTGAACAG
GCCAIIII
41C
I = inosine (universal base)

FIG. 18A

| Cond | L1 Oligo | Splint | L2 Oligo | Splint Annealing | Ligation Temp |
|---|---|---|---|---|---|
| 1 | Bio_P5_i1a_ksplint | Kangaroo splint | Std L2 SL Oligo | Slow | 20C & 12C |
| 2 | Bio_P5_i1a_ksplint | Kangaroo splint | Std L2 SL Oligo | 75C>Ice | |
| 3 | Bio_P5_i1a_ksplint | 6bp-New_8bp-K Splint | L2_6bp SL Oligo | | |
| 4 | Bio_P5_i1a_ksplint | 4bp-New_8bp-K Spint | L2_6bp SL Oligo | | |
| 5 | Bio_P5_i1a_ksplint | 6bp-New_6bp-K Spint | L2_6bp SL Oligo | | |
| 6 | Bio_P5_i1a_ksplint | 1i6bp-New_8bp-K Splint | L2_6bp SL Oligo | | |
| 7 | Bio_P5_i1a_ksplint | 2i6bp-New-8bp-K Splint | L2_6bp SL Oligo | | |
| 8 | Bio_P5_i1a_ksplint | 2i4bp-New-8bp-K Splint | L2_6bp SL Oligo | | |
| 9 | Bio_P5_i1a_ksplint | 4i4bp-New-8bp-K Splint | L2_6bp SL Oligo | | |

FIG. 18B

Normalized Average Ligation Efficiency
Total FL/Toal FL Cntrl
1.40
1.20
1.00
0.80
0.60
0.40
0.20
0.00
1.00
1.04
0.82
0.55
0.13
0.81
1.03
0.64
0.62
1.12
1.17
0.90
0.63
0.07
0.80
0.85
0.46
0.53
Kangaroo splint (20C)
Kangaroo splint, Slow ANL, (20C)
4bp-New_8bp-K-Splint (20C)
6bp-New_6bp-K Splint (20C)
1i6bp-New_8bp-K-Splint (20C)
2i6bp-New-8bp-K Splint (20C)
2i4bp-New-8bp-K Splint (20C)
4i4bp-New-8bp-K Splint (20C)
Kangaroo splint (12C)
Kangaroo splint, Slow ANL, (12C)
4bp-New_8bp-K-Splint (12C)
6bp-New_6bp-K Splint (12C)
1i6bp-New_8bp-K-Splint (12C)
2i6bp-New-8bp-K Splint (12C)
2i4bp-New-8bp-K Splint (12C)
4i4bp-New-8bp-K Splint (12C)
20C ligation
12C ligation

FIG. 18C

Double-Stranded Splint Ligation
5′
P5
Index1
KS-3
ANNEAL
5′
P5
Index1
KS-3′
5′
Index2
MS-3′
KS-3
Index2′
MS-3
5′
LIGATE
5′
P5
Index1
KS-3′
Index2
MS-3′
KS-3
Index2′
MS-3
5′
DENATURE
5′
P5
Index1
KS-3′
Index2
MS-3′
PERFORM 2ND ROUND
5′
P5
Index1
KS-3′
Index2
MS-3′
Index3
Hyb

FIG. 19A

1st level ddbiotin oligo binding
Wash M280 beads
Bind ddbiotin oligos to beads
Wash beads individually
Pool the beads
2nd level indexed oligo ligation
Add ligation master mix to bead pool
Distribute the bead pool
Add dsindex oligos to each well
Heat and anneal the dsindex oligos
Ligation
Inactivate ligase with EDTA
Pool the beads
Wash
Denature the bottom strand
Wash
3rd level indexed oligo ligation
Add ligation master mix to bead pool
Distribute the bead pool
Add dsindex oligos to each well
Heat and anneal the dsindex oligos
Ligation
Inactivate ligase with EDTA
Pool the beads
Wash
Denature the bottom strand
Wash
Resuspend in 500mM NaCl wash buffer

FIG. 19B

Europäisches
Patentamt
European
Patent Office
Office européen
des brevets

# EUROPEAN SEARCH REPORT

Application Number
EP 26 17 9466

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/060830 A1 (PLEXIUM INC [US]; ROKICKI JOSEPH FRANKLIN [US] ET AL.) 28 March 2019 (2019-03-28) * figure 7 * ----- | 1-15 | INV. C12Q1/6806 C12N15/10 C12Q1/6834 |
| X | WO 2020/069386 A2 (CALIFORNIA INST OF TECHN [US]) 2 April 2020 (2020-04-02) * claim 1; figure 1A; example 2 * ----- | 1-15 | |
| X | US 2018/363029 A1 (HINDSON BENJAMIN [US] ET AL) 20 December 2018 (2018-12-20) * paragraph [0105]; figures 1A, 3B * ----- | 1-15 | |
| X | WO 2016/138500 A1 (CELLULAR RES INC [US]) 1 September 2016 (2016-09-01) * paragraph [0177]; claim 1; figures 14-16 * ----- | 1-15 | |
| | | | TECHNICAL FIELDS SEARCHED (IPC) |
| | | | C12Q |

The present search report has been drawn up for all claims

| Place of search | Date of completion of the search | Examiner |
|---|---|---|
| The Hague | 12 August 2026 | Aslund, Fredrik |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# ANNEX TO THE EUROPEAN SEARCH REPORT ON EUROPEAN PATENT APPLICATION NO.

EP 26 17 9466

This annex lists the patent family members relating to the patent documents cited in the above-mentioned European search report. The members are as contained in the European Patent Office EDP file on
The European Patent Office is in no way liable for these particulars which are merely given for the purpose of information.

12-08-2026

| Patent document cited in search report | Publication date | Patent family member(s) | Publication date |
|---|---|---|---|
| WO 2019060830 A1 | 28-03-2019 | CA 3076747 A1 | 28-03-2019 |
| | | CN 111971124 A | 20-11-2020 |
| | | CN 115506034 A | 23-12-2022 |
| | | EP 3687687 A1 | 05-08-2020 |
| | | IL 273511 A | 31-05-2020 |
| | | IL 330321 A | 01-08-2026 |
| | | JP 7361700 B2 | 16-10-2023 |
| | | JP 2020534872 A | 03-12-2020 |
| | | JP 2024001119 A | 09-01-2024 |
| | | JP 2026062913 A | 10-04-2026 |
| | | KR 20200120602 A | 21-10-2020 |
| | | KR 20240091184 A | 21-06-2024 |
| | | US 2019093103 A1 | 28-03-2019 |
| | | US 2019210018 A1 | 11-07-2019 |
| | | US 2019358629 A1 | 28-11-2019 |
| | | US 2020001295 A1 | 02-01-2020 |
| | | US 2020324287 A1 | 15-10-2020 |
| | | US 2021394178 A1 | 23-12-2021 |
| | | US 2022025358 A1 | 27-01-2022 |
| | | US 2023234065 A1 | 27-07-2023 |
| | | WO 2019060830 A1 | 28-03-2019 |
| | | WO 2019060857 A1 | 28-03-2019 |
| WO 2020069386 A2 | 02-04-2020 | US 2020102556 A1 | 02-04-2020 |
| | | WO 2020069386 A2 | 02-04-2020 |
| US 2018363029 A1 | 20-12-2018 | NONE | |
| WO 2016138500 A1 | 01-09-2016 | CN 107208157 A | 26-09-2017 |
| | | EP 3262189 A1 | 03-01-2018 |
| | | ES 2906221 T3 | 13-04-2022 |
| | | US 2016257993 A1 | 08-09-2016 |
| | | US 2024318227 A1 | 26-09-2024 |
| | | WO 2016138500 A1 | 01-09-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 63214693
- WO 2020086746 A
- WO 2007010251 A
- WO 2006064199 A
- WO 2005065814 A
- WO 2015106941 A
- WO 1998044151 A
- WO 2000018957 A
- US 20180023119 A
- US 20180355348 A
- WO 04018497 A
- US 7057026 B
- US 7329492 B
- US 7211414 B
- US 7315019 B
- US 7405281 B
- US 20180127816 A
- US 20210087613 A
- US 9441267 B
- US 9045796 B


### Non-patent literature cited in the description


- **BENTLEY et al.** *Nature*, 2008, vol. 456, 53-59